# EUROPEAN PATENT SPECIFICATION

(11) **EP 3 890 746 B1**
(45) Date of publication and mention of the grant of the patent: **04.02.2026**
(21) Application number: 19892028.2
(22) Date of filing: 06.12.2019
(51) Int. Cl.: A61K 31/593, A61K 9/107, A61K 9/127, C07F 9/10, A61P 27/02, A61K 47/24

(54) **VITAMIN D MICRO-EMULSIONS AND USES THEREOF**
VITAMIN-D-MIKROEMULSIONEN UND DEREN VERWENDUNGEN
MICRO-ÉMULSIONS DE VITAMINE D ET LEURS UTILISATIONS

(30) Priority: 06.12.2018 US 201862776022 P
(43) Date of publication of application: 13.10.2021
(73) Proprietor: Chemipal Global Entrepreneurship Ltd., Netanya 4250432 (IL)
(72) Inventor: EZRA, Rafael, 6233121 Tel-aviv (IL); MANOR, Yoni, 34404 Haifa (IL)
(74) Representative: Latscha Schöllhorn Partner AG
(86) International application number: PCT/IB2019/001314
(87) International publication number: WO 2020/115552

(56) References cited:
- WO-A1-94/01089
- WO-A1-97/18817
- WO-A2-2018/209293
- CN-A- 105 535 021
- DE-A1- 19 519 273
- US-A1- 2005 026 877
- US-A1- 2011 015 266
- US-A1- 2011 136 912
- US-A1- 2018 008 538
- BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631 - 662, XP009102859, ISSN: 0193-2691, DOI: 10.1081/DIS-120015368
- FAHIM M. M. ET AL.: "Fluorophotometry as a diagnostic tool for the evaluation of dry eye disease", BMC OPHTHALMOLOGY, vol. 6, 20, XP021016328, DOI: 10.1186/1471-2415-6-20
- ORLOVA T.: "Possible use of provitamin D3 photoisomerization for spectral dosimetryof bioactive antirachitic UV radiation", JOURNAL OF APPLIED SPECTROSCOPY, vol. 76, no. 2, 2009, pages 240 - 244, XP55751257, DOI: 10.1007/s10812-009-9159-1

## Description

### FIELD OF THE INVENTION

Pharmaceutical compositions designed to deliver lipophilic agents (e.g., drugs molecules and nutrients) efficiently to the eyes and methods for using same are described herein. More particularly, ophthalmic pharmaceutical compositions for use in the treatment of disorders of the anterior and posterior segments of the eye and for maintenance of a healthy ocular state are envisioned. Ophthalmic pharmaceutical compositions described herein may also be used to slow the aging process of the eye by, for example, efficient delivery of nutrients (e.g., Vitamin D) to the eye that confer healthful benefits thereto. The composition may also be used as a medical device which once on the surface of the eye, creates a layer that filters electro-magnetic radiation in different wave lengths. The composition may also be used as a medical device which eases the symptoms and signs of eye-surface conditions.

### BACKGROUND OF THE INVENTION

The eye is protected by a variety of structures at the anterior surface of the eye including the eyelids, conjunctiva, and the cornea. The posterior surfaces of the lids are covered with a mucous membrane and the palpebral conjunctiva which reflects onto the eye to become the bulbar conjunctiva. The bulbar conjunctival epithelium is continuous with the corneal epithelium which accounts for about 10% of the anterior surface of the eye and is where most of the stationary refraction occurs. The corneal epithelium is 4-5 cells thick and the superficial cells therein contain many microvilli, which structures aid in maintaining the moisture of the epithelial surface by promoting the adhesion of the tear film. The tear film lubricates the anterior surface of the eye thereby decreasing the frictional forces arising from the blinking of the eyelids, the presence of foreign particles on the surface of the eye, and rotational movements of the eyeball. The tear film also provides a medium that transfers oxygen from the ambient air to the cornea.

The anterior surface of the eye is vulnerable to damage inflicted by various insults due to mechanical abrasion of the cornea; contact lens use; spontaneous peeling of the epithelium; damaged epithelium and stroma following photo-refractive keratectomy; chemical burns; over exposure to ultraviolet light including sunlight; systemic diseases such as Sjogren syndrome, Steven-Johnson syndrome, Cicatricial pemphigoid syndrome; chronic edema of cornea with recurrent erosion of epithelium; impaired tear film formation, and conditions resulting from epithelia damaged by radial keratotomy.

Aging is also associated with disorders resulting from slow regeneration of the epithelium. Ageassociated impaired regeneration and cellular abnormalities can cause thinning of the epithelial layer and impaired adherence of the epithelial layer to the basal lamina, thus decreasing the ability of the cornea to retain the tear film, which in turn leads to further epithelial damage.

Following injury to the corneal epithelium, nearby cells retract slightly, round up and begin an ameboid migration from the basal layer across the exposed basement membrane to cover the defect with a new monolayer of cells. These cells then take on the characteristics of a new basal layer and undergo mitosis to gradually fill in the defect with the full complement of four to five layers of cells. Present treatment for corneal wounds involves applying eye drops to the surface in order to protect the delicate healing process from erosion due to blinking and the other sources of mechanical friction. There are no currently used medicaments that promote the healing process itself.

Electro-magnetic radiation in different wave lengths is a generator of damage to both surface and interior chambers of the eye. UV radiation and blue-light have both been documented to create damage to the tissues and generate high oxidative load.

Certain compositions of Vitamin D and phospholipids and their potential ophthalmic and topical applications were previously described. CN 105 535 021, WO 94/01089 and DE 195 19 273 described certain liposomal compositions of this type, which are essentially different from nano-emulsions by structural, and as a result, their functional properties. WO 97/18817 described certain eye drop formulations of vitamin D, which are not phospholipid nano-emulsions and therefore lack light absorbance properties outside the UV range. US 2005/026877 described certain submicronic emulsions of Vit D and hydrophilic surfactants, but not their light blocking effects. US 2011/136912 described submicronic emulsions with a charged phospholipid component and their use for ocular delivery of therapeutic actives, including among others various types of vitamins.

### SUMMARY OF THE INVENTION

Covered embodiments are defined by the claims, not this summary. This summary is a high-level overview of various aspects and introduces some of the concepts that are further described in the Detailed Description section below. This summary is not intended to identify key or essential features of the claimed subject matter, nor is it intended to be used in isolation to determine the scope of the claimed subject matter. The subject matter should be understood by reference to appropriate portions of the entire specification, any or all drawings, and each claim.

The present invention is based at least in part on the surprising discovery that nano-emulsions described herein can act as an effective energetic light filter at the near visible spectral rage, which facilitates and enhances the protection of the different eye components from its harmful effect. The significance of this discovery is underscored by the unexpected discovery that the nano-emulsions described herein facilitate the protection of the eye components with minimal to no effect on the visible light spectral range.

Results presented herein reveal that the surprising properties of nano-emulsion formulations described herein incorporate a lipophilic agent, specifically Vitamin D known to be irritating to the eye, demonstrating it can be tailored to maximize delivery of particular lipophilic agents (e.g., drugs molecules and nutrients) to the eye, not generally considered tolerable by patients, especially for prolonged duration.

In a particular embodiment, nano-emulsions comprising phospholipids described herein exhibit the following innovative properties:
- Phospholipid nano-emulsions facilitate longer contact time on the surface of the cornea as compared to any other forms of eye-drops. The longer contact time improves diffusion of drug molecules into epithelial cells of the cornea, thereby improving efficacy of the drug treatment.
- Phospholipid nano-emulsions facilitate closer contact between oily, non-polar drug molecules and the polar surface of the corneal epithelium cells. In contrast, close contact cannot be achieved with oily eye drops because the physiological water phase forms a polar barrier, preventing the approach of the oily molecules. The presence of the phospholipids in the nano-emulsions creates a localized environment wherein the oily molecules can be immersed in the water phase, thereby coming into closer contact with the cornea and improving the uptake of the oily molecules into the corneal cells. The unexpected effect of nano-emulsions described herein is due at least in part to the localized microenvironment that the phospholipid nano-emulsion creates over the surface of the eye and between the oil and water phases. The stable structure of oily phase over a water phase is supplemented and enhanced by the presence of immersed oily molecules within the water phase. See, for example, [0031].

In an aspect, an ophthalmic topical pharmaceutical composition comprising Vitamin D in a phospholipid nano-emulsion is presented, wherein the Vitamin D ranges from 0.02-2% w/v, preferably at about 0.1% w/v, of the pharmaceutical composition: wherein emulsified particles in the phospholipid nano-emulsion have a particle size ranging from 100-300 nanometers in diameter; wherein phospholipids and vitamin D in the pharmaceutical composition are in a ratio of at least 3:1 and wherein the phospholipids range from 0.1-5% w/v, preferably at about 0.3% w/v; and wherein a therapeutically effective amount of the pharmaceutical composition is sufficient to absorb light in a wavelength range of 220-520nm. In an embodiment thereof, the Vitamin D ranges from 0.05-0.5% of the phospholipid nano-emulsion. In a further embodiment thereof, the Vitamin D is present at about 1000 µg per 1ml of the phospholipid nano-emulsion or is present at about 100 µg per 1ml. In a further embodiment thereof, the wavelength range comprises 230-520nm. In more particular embodiments, the wavelength range comprises 280-320nm; the wavelength range comprises 320-400nm; the wavelength range comprises 400-495nm; the wavelength range comprises 415-455nm; or the wavelength range comprises 280-315nm. In an embodiment thereof, the light absorbing is measured using a spectrophotometer. In a further embodiment thereof, the pharmaceutical composition does not comprise a liquefied or compressed gas propellant.

In an embodiment thereof, the pharmaceutical composition is for use in reducing absorption of light in a wavelength range of 220-520nm in eyes of a subject in need thereof. In a further embodiment thereof, the pharmaceutical composition is for use in reducing absorption of light in a wavelength range of 220-520nm in eyes of a subject in need thereof, wherein the pharmaceutical composition is administrable onto the corneal tissue of the eyes of the subject.

In an embodiment thereof, the pharmaceutical composition is for use in reducing absorption of light in a wavelength range of 220-500nm in eyes of a subject in need thereof. In a further embodiment thereof, the pharmaceutical composition is for use in reducing absorption of light in a wavelength range of 220-500nm in eyes of a subject in need thereof, wherein the pharmaceutical composition is administrable onto the corneal tissue of the eyes of the subject.

In another embodiment thereof, the pharmaceutical composition is for use in treating a condition of the eye in a subject in need thereof. In a further embodiment thereof, the condition of the eye comprises at least one of inflammation of the eye, dry eye, or glaucoma.

In another embodiment thereof, the pharmaceutical composition is for use in the preparation of a medicament for reducing absorption of light in a wavelength range of 220-520nm in eyes of a subject in need thereof. In a further embodiment thereof, the pharmaceutical composition is for use in the preparation of a medicament for reducing absorption of light in a wavelength range of 220-500nm in eyes of a subject in need thereof.

In another aspect, a pharmaceutical composition comprising Vitamin D in a phospholipid nano-emulsion is presented, wherein the Vitamin D ranges from 0.02-2% wt/vol or 0.07-1.4% wt/vol of the phospholipid nano-emulsion in the pharmaceutical composition: wherein emulsified particles in the phospholipid nano-emulsion have a particle size ranging from 100-300 nanometers in diameter; wherein phospholipids and Vitamin D in the pharmaceutical composition are in a ratio of at least 3:1 and wherein the phospholipids range from 0.1-5% w/v, preferably at about 0.3% w/v; and wherein a therapeutically effective amount of the pharmaceutical composition is sufficient to deliver the Vitamin D to corneal tissue of an eye for a prolonged duration, wherein the prolonged duration is at least 1-10 minutes. In an embodiment thereof, the prolonged duration is determined by fluorophotometry. In a further embodiment thereof, the prolonged duration is at least one minute. In a still further embodiment thereof, the prolonged duration is at least one minute and less than 12 minutes. In another embodiment thereof, the pharmaceutical composition does not comprise a liquefied or compressed gas propellant.

In an embodiment thereof, the pharmaceutical composition is for use in delivering the Vitamin D to eyes of a subject in need thereof. In a more embodiment thereof, the pharmaceutical composition is for use in delivering the Vitamin D to the eyes of a subject in need thereof, wherein the pharmaceutical composition is administrable onto the corneal tissue of the eyes of the subject.

In an embodiment thereof, the pharmaceutical composition is for use in treating a condition of the eye in a subject in need thereof. In an embodiment thereof, the condition of the eye comprises at least one of inflammation of the eye, dry eye, or glaucoma.

In an embodiment thereof, the pharmaceutical composition is for use in the preparation of a medicament for the treatment of a condition of the eye in a subject in need thereof. In an embodiment thereof, the condition of the eye comprises at least one of inflammation of the eye, dry eye, or glaucoma.

In an embodiment thereof, the pharmaceutical composition is configured to deliver the Vitamin D to the corneal tissue with minimal irritation to corneal tissue.

In an embodiment thereof, the pharmaceutical composition is incorporated into a medical device.

In another aspect, a method for reducing exposure of a subject's eyes to light is presented, wherein the light comprises a wavelength range of 220-520nm, the method comprising: contacting corneal tissue of the eye with the pharmaceutical composition, wherein a therapeutically effective amount of the pharmaceutical composition is sufficient to absorb light comprising a wavelength range of 220-520nm. In an embodiment, the method further comprises exposing the subject's eyes to light comprising a wavelength range of 220-520nm, wherein the contacting reduces the amount of the light comprising the wavelength range of 220-520nm absorbed by the corneal tissue of the subject's eyes contacted with the pharmaceutical composition relative to that of corneal tissue of the subject's eyes prior to contacting with the pharmaceutical composition, thereby reducing exposure of the corneal tissue of the subject's eyes contacted with the pharmaceutical composition to the light comprising the wavelength range of 220-520nm. In a further embodiment, the amount of light is determined using a nanodrops spectral absorption meter.

In another aspect, the pharmaceutical composition for use in a method for treating an eye condition in a subject in need thereof is presented, the method comprising: administering the pharmaceutical composition to corneal tissue of the subject's eyes, wherein a therapeutically effective amount of the pharmaceutical composition is sufficient to treat the eye condition in the subject. In an embodiment, the condition of the eye is at least one of an inflammation of the eye, dry eye, or glaucoma.

In a particular embodiment, phospholipid nano-emulsions comprising Vitamin D are envisioned. Vitamin D is a particularly challenging agent to deliver to the eye for a variety of reasons. At the outset, it is challenging because Vitamin D is irritating to the eye surface. Accordingly, patients are typically not willing to endure the discomfort associated with administering Vitamin D in any form to the eye. Delivery of Vitamin D to the eye is also problematic because this nutrient is an oily molecule and as such, has a very short retention time on the surface of the eye due to the natural aqueous environment of the eye's surface and the polar nature of the corneal epithelium. The short retention time of vitamin D on the surface of the eye, in turn, makes it difficult to achieve a minimum therapeutic or otherwise concentration of Vitamin D at the eye surface.

Phospholipid nano-emulsions comprising Vitamin D circumvent the aforementioned challenges associated with delivery of Vitamin D to the eye. Unexpectedly, when Vitamin D was administered to the eye in a phospholipid nano-emulsion, no irritation was detectable. See Tables 1-6 below, which demonstrate that Vitamin D eye drops group (Vitamin D-Lipitear formulation; Test Item 1) does not cause irritation when administered to an animal model in which eye irritation is routinely assessed. Further, when Vitamin D was formulated into a phospholipid nano-emulsion, the retention time on the eye surface was prolonged significantly. These findings demonstrate that the phospholipid nano-emulsions make administration of Vitamin D via topical application directly to the eye a viable therapeutic regimen. Vitamin D in the form of phospholipid nano-emulsions penetrates the cornea and the interior chamber of the eye to a surprising degree, the present discoveries also demonstrate that therapeutically effective amounts of vitamin D can be delivered to the eye via topical administration. These features are due at least in part to the increased retention time and closer contact with the corneal epithelium made possible by phospholipid nano-emulsions described herein.

The present phospholipid nano-emulsions provide rapid relief from symptoms associated with ocular Vitamin D deficiency and efficient delivery of Vitamin D.

In a particular embodiment, phospholipid nano-emulsions comprising Vitamin D create a surprising physical ability to filter electro-magnetic radiation in various wave lengths. The Vitamin D phospholipids nano-emulsion, once administered to the eye and thus, placed over the eye filtered Blue light and UV radiation. This property is surprising not just in the quality aspect but also quantitatively since the thickness of the Vitamin D Nano-emulsion on the surface of the eye corresponds to the thickness of the tear film and yet, the ability to filter the electro-magnetic radiation has been demonstrated.

The invention now will be illustrated with reference to some non-limiting drawings and examples.

### BRIEF DESCRIPTION OF THE DRAWINGS

Some embodiments of the disclosure are herein described, by way of example only, with reference to the accompanying drawings. With specific reference now to the drawings in detail, it is stressed that the embodiments shown are by way of example and for purposes of illustrative discussion of embodiments of the disclosure. In this regard, the description taken with the drawings makes apparent to those skilled in the art how embodiments of the disclosure may be practiced.

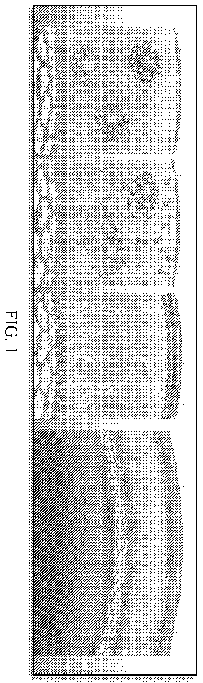

FIG 1. presents a cartoon depicting how nano-emulsion compositions described herein form a dynamic structure comprising three layers on the surface of the eye. The uppermost layer of the nano-emulsion composition/formulation, which is furthest from the cornea, comprises the oily phase; the middle layer comprises the phospholipids; and the lower layer, which is in contact with the cornea, comprises mainly water.

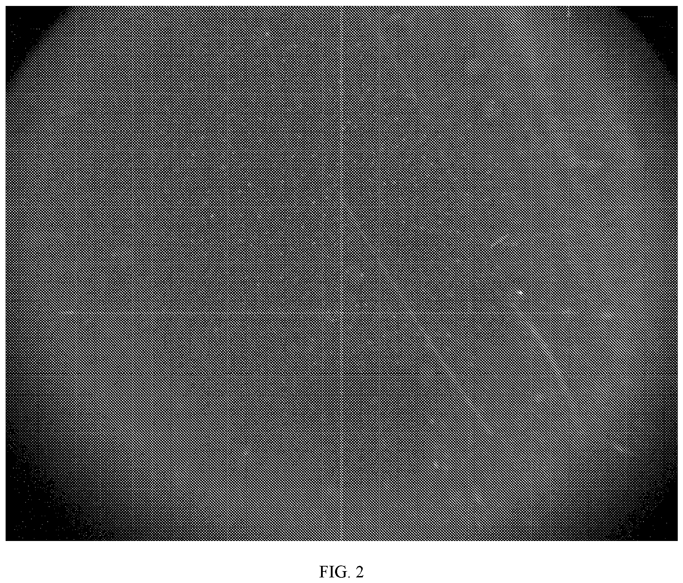

FIG 2. is a photograph of a microscope slide on which a tear-film thick phospholipid containing nano-emulsion comprising Vitamin D has been placed.

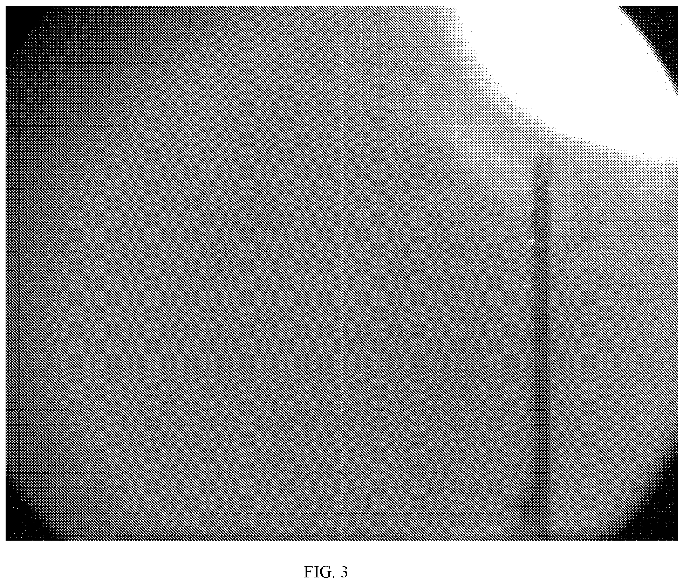

FIG 3. is a photograph of a microscope slide on which a tear-film thick phospholipid containing nano-emulsion comprising Vitamin D has been placed.

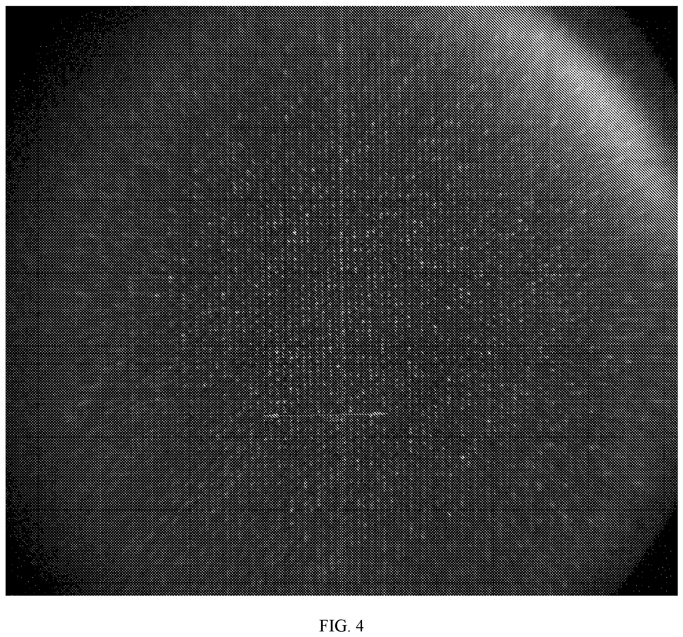

FIG 4. is a photograph of a microscope slide on which a tear-film thick phospholipid containing nano-emulsion comprising Vitamin D has been placed, depicting a reference resolution of 20 lines of 60 nanometers in width, over a 1200 nanometer line, as observed at 40X magnification.

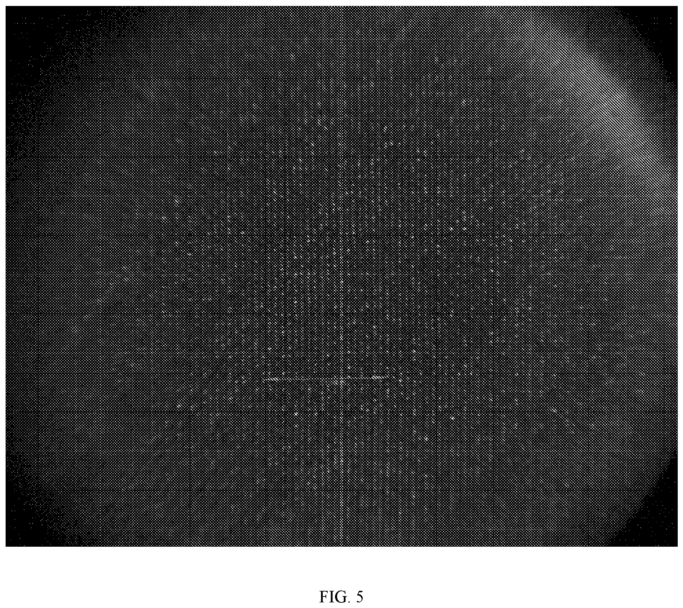

FIG 5. is a photograph depicting resolution through a tear-film thick phospholipid containing nano-emulsion comprising Vitamin D as described herein.

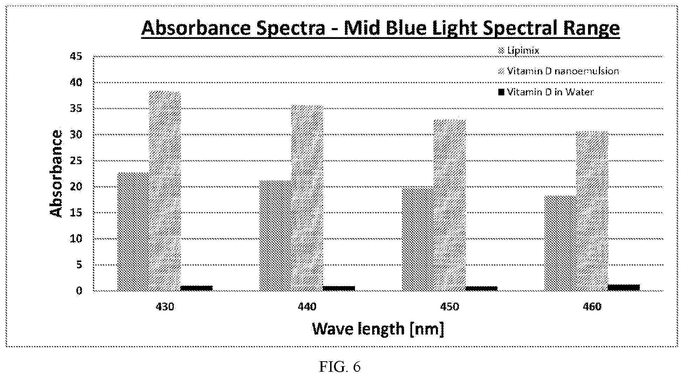

FIG 6. is a histogram plot depicting absorbance properties of Vitamin D in water, Lipimix alone, or Vitamin D nano-emulsion (Vitamin D in Lipimix) in the blue light spectral range as measured through a tear-film thick layer.
FIG 7. is a line graph depicting absorbance properties of Vitamin D in water, Lipimix alone, or Vitamin D nano-emulsion (Vitamin D in Lipimix) in the blue light spectral range as measured through a tear-film thick layer.
FIG 8. is a line graph depicting absorbance properties of Vitamin D in water, Lipimix alone, or Vitamin D nano-emulsion (Vitamin D in Lipimix) in the UVB (280-315nm) spectral range as measured through a tear-film thick layer.
FIG 9. is a line graph depicting absorbance properties of different concentrations of Vitamin D nano-emulsion or nano-emulsion alone in the UV to blue light spectral range as measured through a tear-film thick layer.
FIG 10. is a line graph depicting absorbance properties of different concentrations of Vitamin D nano-emulsion or nano-emulsion alone in the UV to blue light spectral range as measured through a tear-film thick layer. The concentrations are X5 lower relative to those used with respect to 00 to simulate conditions on the surface of the eye.
FIG 11. is a line graph depicting absorbance properties of Vitamin D in water, Lipimix alone, or Vitamin D nano-emulsion (Vitamin D in Lipimix) in the UV to blue light spectral range performed in parallel with assays depicted in [0038] and 0.
FIG 12. A-C present line graphs depicting absorbance properties of different concentrations of Vitamin D nano-emulsion or nano-emulsion alone in the (A) UVB range; (B) UVA range; and (C) blue light spectral range. The different concentrations of Vitamin D nanoemulsion are the same as those depicted in 0.

### DETAILED DESCRIPTION OF THE INVENTION

Among those benefits and improvements that have been disclosed, other objects and advantages of this disclosure will become apparent from the following description taken in conjunction with the accompanying figures. Detailed embodiments of the present disclosure are disclosed herein; however, it is to be understood that the disclosed embodiments are merely illustrative of the disclosure that may be embodied in various forms. In addition, each of the examples given regarding the various embodiments of the disclosure which are intended to be illustrative, and not restrictive.

Throughout the specification and claims, the following terms take the meanings explicitly associated herein, unless the context clearly dictates otherwise. The phrases "in one embodiment," "in an embodiment," and "in some embodiments" as used herein do not necessarily refer to the same embodiment(s), though it may. Furthermore, the phrases "in another embodiment" and "in some other embodiments" as used herein do not necessarily refer to a different embodiment, although it may. All embodiments of the disclosure are intended to be combinable without departing from the scope or spirit of the disclosure.

As used herein, the term "based on" is not exclusive and allows for being based on additional factors not described, unless the context clearly dictates otherwise. **In** addition, throughout the specification, the meaning of "a," "an," and "the" include plural references. The meaning of "in" includes "in" and "on."

The cornea is an avascular organ that obtains nutrition from the vasculature of the limbus by diffusion. It is, therefore, vulnerable to nutrient deprivation in light of its physiologically isolated situation. Moreover, in that the cornea is a semi-rigid structure comprising cells that are tightly adhered to form a relatively impermeable tissue, it is a challenging barrier for pharmacological intervention. Indeed, the cornea presents a barrier into which it is difficult to diffuse and through which it is even more difficult to traverse. Delivery of lipophilic drug molecules to the cornea is particularly impaired by the polar nature of the corneal epithelium environment. As a result, lipophilic molecules are impaired with respect to their ability to encounter epithelial cells at the anterior segment of the eye and thus, even less likely to be able to exert a pharmacological effect in these cells. Additional pharmacological challenges arise from poor absorption of lipophilic drugs through the anterior segment of the eye, which reduces the chances for achieving a minimum active concentration of a lipophilic drug at the posterior segment. The combined impact of the above challenges for delivering lipophilic drugs to the eye is that the likelihood of achieving pharmacological effects at the posterior segment of the eye is vanishingly small.

Emulsions are drug vehicles used in a variety of pharmaceutical preparations. The main pharmaceutical advantage of emulsions is their ability to combine an oil phase with an aqueous/water medium wherein the surface tension between the oil and the water is maintained by surfactants. In recent years new techniques have been developed to produce nano-emulsions in which the oily droplets are less than 300 nanometers in size. The particle size in nano-emulsions offers a more stable thermodynamic condition as compared to regular emulsions. Pharmaceutically, nano-emulsion technology offers an opportunity to formulate topical emulsions of drug molecules which were not stable using previously disclosed techniques. As a practical vehicle, nano-emulsions are now used in various preparations for topical application.

The present inventors have discovered that particular nano-emulsion formulations described herein can surprisingly be used as a drug delivery system for delivering lipophilic drugs and nutrients (e.g., Vitamin D) to the eye. The unexpected property of these nanoemulsion formulations confers the ability to deliver lipophilic drugs and nutrients effectively into and through the corneal barrier. Topical preparations intended for ocular use must take into account the physical properties of the eye and challenges associated therewith. The surface of the cornea is, for example, very smooth and continuously washed by tears and swept by the eyelids. These features minimize retention times during which a therapeutic agent or nutrient can be absorbed by the corneal epithelium. Moreover, tight junctions between corneal epithelium layers render the cornea relatively impermeable, making it almost impossible for drug molecules to diffuse into the cornea. Additional challenges arise from the lipophobic structure of the cornea. The aforementioned physical properties of the cornea make diffusion through the cornea to reach posterior segments of the eye even more problematic.

Results presented herein reveal that the surprising properties of nano-emulsion formulations described herein can be tailored to maximize delivery of particular lipophilic agents (e.g., drugs molecules and nutrients) to the eye, even lipophilic agents that are known to be irritants to the eye and thus, not generally considered tolerable by patients, especially for prolonged duration. An exemplary lipophilic agent for which nano-emulsion formulations described herein have been optimized is Vitamin D.

Unique and surprising properties of nano-emulsion formulations described herein facilitate longer corneal retention time, enhanced stable contact between lipophilic agents and the cornea, and enhanced corneal penetration. With respect to longer corneal retention time, nanoemulsion formulations described herein mimic the behavior of the lipid fraction of the natural tear film, thereby maintaining contact between a lipophilic agent in the formulation and the cornea for durations of up to a few minutes. This stands in marked contrast to standard eye drop formulations comprising drug molecules that typically remain in contact with the cornea for only a few seconds. Indeed, the present nano-emulsion formulations have been designed to exhibit prolonged retention times for contact with the cornea that match the tear-film lipid phase of ten minutes. Accordingly, nano-emulsion formulations described herein provide therapy for prolonged duration sufficient to improve diffusion of lipophilic agents into the epithelial layers of the cornea and into the posterior segment of the eye. These properties maximize the therapeutic efficacy of nano-emulsion formulations comprising lipophilic agents described herein.

With respect to providing enhanced stable contact between lipophilic agents and the cornea, nano-emulsion formulations described herein form a dynamic structure of three layers when administered to an eye. See [0031]. The uppermost layer of the nano-emulsion formulation, which is furthest from the cornea, comprises the oily phase; the middle layer comprises the phospholipids; and the lower layer, which is in contact with the cornea, comprises mainly water. Accordingly, the aqueous/water layer is in direct contact with the cornea and serves as a reservoir in which the corneal epithelial cells are bathed. A drug molecule must, therefore, be present in the aqueous/water layer in a sufficient concentration to achieve absorption levels necessary to achieve therapeutic efficacy. Since lipophilic molecules are thermodynamically predisposed to adhere to the upper oily environment, they are typically excluded from the aqueous/water layer, thereby impairing their delivery to the corneal epithelial cells. The nano-emulsion formulations described herein overcome this impediment to delivery by creating a dynamic structure in which spheres of lipophilic agents (e.g., drug molecules or nutrients) surrounded by phospholipids continuously cycle into the aqueous/water layer and deliver lipophilic molecules therein in close proximity to the epithelium cells. This structural/functional feature of the nano-emulsion formulations described herein creates a dynamic equilibrium that facilitates efficient delivery of lipophilic molecules to the corneal epithelium cells and absorption thereby.

With respect to providing enhanced corneal penetration, nano-emulsion formulations described herein are specifically designed to penetrate the sclera and cornea. The sclera is nearly impermeable and the cornea is highly resistant to diffusion due to the presence of specialized tight junctions between corneal cells. When used at suitable concentrations and ratios, the phospholipid components of the present nano-emulsion formulations surprisingly act as penetration enhancers that promote diffusion of lipophilic molecules into and across the cornea, thereby providing better absorption of the lipophilic molecules and resultant improved therapeutic efficacy.

The combined effect of longer corneal retention time, enhanced stable contact between lipophilic agents and the cornea, and enhanced corneal penetration confers upon nano-emulsion formulations described herein definitively greater absorption of lipophilic molecules into the anterior and posterior segments of the eye. Accordingly, these nano-emulsion formulations provide an effective ocular drug delivery system for lipophilic drugs and lipophilic nutrients. Further to the above, pharmaceutical compositions for the treatment of disorders of the anterior and posterior segment of the eye are presented comprising at least one lipophilic agent (e.g., a lipophilic drug or lipophilic nutrient), phospholipids, and an ophthalmologically acceptable carrier.

The term "treatment" refers to alleviation of some of the undesired symptoms of various eye disorders, and/or to curing the disorder of the eye. Nano-emulsion formulations and/or compositions may also be used to maintain normal health of the eye.

The term "anterior segment of the eye" refers to the corneal and conjunctival epithelium and includes the epithelial cells, as well as the glands present in the epithelium.

The term "disorders of the anterior segment of the eye" refers to disorders which cause or result from physical damage to at least one of the corneal or conjunctival epithelium, decreased regeneration rate for cells comprising the corneal or conjunctival epithelium, and diminished secretions from glands present in the conjunctival epithelium. In order to treat disorders of the anterior segment of the eye, one must achieve at least a minimal concentration wherein an active pharmaceutical ingredient (API) is pharmacologically effective in the anterior segment of the eye.

Typical disorders of the anterior segment of the eye caused by physical or chemical damage result from mechanical abrasion of the cornea, corneal epithelial defects created by wearing contact lenses, corneal epithelial defects created by spontaneous peeling of the epithelium, corneal damage following photoreactive keratectomy, injuries caused by chemical substances, damage caused by exposure to ultraviolet light, systemic diseases that damage the corneal epithelium and conjunctiva, for example, Sjorgren-Syndrome, Steven-Johnson Syndrome, Cicatricial Pemphigoid Syndrome, chronic edema of the cornea with recurrent erosion of epithelium and the like.

Typical disorders of the anterior segment of the eye caused by a decrease in the regenerative rate of cells include deterioration of the eye due to old age or an anti-proliferative treatment. The pharmaceutical composition of the present invention may be administered to persons suffering from disorders which cause damage to the corneal or conjunctival epithelia, or in conjunction with treatments which are known to cause such damage, for example, laser or radial keratectomy or administration of various systemic or topical medications.

The term "phospholipids" refers to a class of lipids comprising a glycerol or sphingosine platform to which is attached one or more fatty acids and a phosphate group to which an alcohol is linked. Naturally occurring phospholipids include, for example, phosphatidylcholine, phosphatidylserine and phosphatidylinositol.

In a particular embodiment, the oily phase comprises medium chain triglycerides and/or vegetable oil including but not limited to soybean oil. In a more particular embodiment, the oily phase consists of medium chain triglycerides and soybean oil.

Pharmaceutical formulations or compositions described herein comprise an oily phase in the range of 0.1-20%, more particularly 0.2-10%. The concentration of phospholipids should be in the range of 0.01-10% weight per volume. In a particular embodiment, phospholipids comprise 12% of the oily phase. The concentration of the active pharmaceutical ingredient may be as little as 0.0001% weight per volume. Specific ratios of oil phase and phospholipids content are adjusted based on the particular active pharmaceutical ingredient being added to the formulation/composition.

Phospholipids used in the pharmaceutical formulations/compositions described herein include, without limitation, phospholipids that are obtainable from commercial providers such as, for example, Lipoid AG. Exemplary phospholipids include E80 and S-75, which may be purchased from Lipoid AG.

### Vitamin D nano-emulsion as UV protectant in the form of eye drops

The damaging effect of direct sun light on human skin are well known and documented. Skin erythema, pigmentations and skin carcinomas are all related to over exposure to direct sun light. However, high energy light and bright light also produces extensive damage to the human eye. Solar retinopathy, Photokeratitis, Punctate Keratitis, and Radiation Cataract are just a few examples of medical conditions attributed to sun light. The main damage is caused by UV radiation (100-400 nm), transferring its energy onto the cornea, lens and retina when absorbed.

Damage to ocular tissue has been demonstrated time and again, more commonly in professional personnel and people who spend a lot of time outside, such as military staff, infrastructure workers, water, snow and all outdoor athletes, who are exposed to direct sun light. UV related eye morbidities is also on the rise and are of considerable concern to physicians and health care systems. According to the 2008 US Army Injury Prevention Report, over 10% of eye injury visits are due to Photokeratitis and Punctate Keratitis, both highly affected by direct sun light. Moreover, studies have shown UV Photokeratitis may appear after only 30 minutes in the sun, which exposes the average person to the threshold dose on a daily basis. Sun glasses do protect the eye quite well, but they possess a limiting effect on eye sight resolution, and their use does not always apply.

As described herein, Vitamin D Nano-emulsion technology reduces the UV radiation which passes through a thin layer resembling the natural tear film on the eye. The formulation eye-drops nano-emulsion reduces the amount of radiation reaching the eyes' tissues. The formulation is effective at the UV-B range, the range most damaging to the eye (280-320 nm). Vitamin D nano emulsions described herein reduce the effective radiation intensity up to 75% at full direct sun exposure. In addition to the direct UV protection, Vitamin D nano emulsions described herein maintain the corneal surface in a hydrated and lubricated state, thus keeping the surface of the eye less susceptible to harsh environmental conditions.

### Blue Light and Direct Ocular Effects

It is well known that use of portable light-emitting devices immediately before bedtime has biological effects that may perpetuate sleep deficiency and disrupt circadian rhythms, both of which can have adverse impacts on performance, health, and safety.

Coupled with the increased usage of computers, there is a concurrent increase in digital eye strain, with around 50% of computer users suffering symptoms which fall into two main categories: those linked to accommodative or binocular vision stress, and those linked to external symptoms linked to dry eye.

The scientific literature shows that the effects of blue light are not limited to eye strain, but that blue light also has direct effects on the eye. The term "blue light hazard" has been coined to describe the danger this light presents to critical structures within the eye and companies focused on prevention are developing technologies to protect the eye from this hazard. High energy blue light can pass through the cornea and lens all the way to the retina, contributing to diseases such as dry eye, cataracts, and age-related macular degeneration, as well as inhibiting melatonin secretion, and enhancing adrenocortical hormone production, among other adverse effects. Researchers have suggested that users should take appropriate protective measures when using blue light-related products, especially at night.

Effects seen include those at the surface of the eye. Researchers have shown that the use of visual display terminals for a short period of time can lower the stability of tear film in volunteers and that the night shift mode may cause less damage to the ocular surface than the normal mode. Researchers concluded that high-energy blue light from visual display terminals can be a risk factor in the ocular surface damage. However, this damage can be reversed.

Much research has been done to determine the mechanisms of blue light induced ocular damage. *In vitro* studies in human corneal epithelial cell studies blue light have shown that blue light increases reactive oxygen species (ROS) production and associated damage. Studies on Dry Eye Disease have shown an association between tear film hyperosmolarity and inflammation severity elicited through ROS induced signaling pathways. The effects of visible light on human corneal epithelial cells and the impact of natural antioxidants on oxidative stress produced by overexposure to light have also been investigated.

Other researchers have investigated the influence of overexposure to light emitting diode (LED)-derived light with various wavelengths on the mouse ocular surface. They concluded that overexposure to blue light with short wavelengths can induce oxidative damage and apoptosis to the cornea, which may manifest as increased ocular surface inflammation and resultant dry eye. Hyperosmolar stress has also been shown to impact phototoxicity, further suggesting that Dry Eye Disease patients might be more sensitive to blue light ocular toxicity. Indeed, dry eye patients have previously been shown to have significant improvement in mean functional visual acuity and visual maintenance ratio whilst wearing eyeglasses with 50% blue light blocked lenses, an effect not seen in healthy controls.

Effects of blue light are not limited to the surface of the eye. Blue light damage to the retina has research support from studies with both acute and chronic exposure. The retina is most sensitive to light at the shorter wavelengths (maximum sensitivity shown at 441 nm). Retinal damage at the shorter visible wavelengths (up to 500 nm) was primarily photochemical in nature (versus purely thermal effects). Retinal photochemical injury from blue light has, furthermore, been demonstrated both *in vitro* and *in vivo. In vitro* studies of human retinal cells have identified cellular signaling pathways that result in oxidative damage which increases the risk of retinal photodamage and contributes to the development of age-related maculopathy. Even at the low intensity utilized in the display devices, blue light can induce ROS production and apoptosis in retinal cells. *In vivo* studies with LED blue light exposure have demonstrated such exposure to cause retinal injury in awake, task-oriented rod-dominant animals. This damage can be induced by commercial light emitting diodes (LEDs), with phototoxicity being characterized by severe damage of photoreceptors and by the induction of necrosis. Further research *in vivo* (rat model) has demonstrated that the blue component of the white-LED may cause retinal toxicity at occupational domestic illuminance and not only in extreme experimental conditions. Current regulations and standards for exposure have been established on the basis of acute light exposure and do not take into account the effects of repeated exposure

Interestingly the blue component of white light has also been shown to cause dysfunction of the blood retinal barrier at the retinal pigment epithelium. Researchers have suggested that a blue light blocking filter should be used in ocular surgery on humans when an operating microscope is being used.

Filters can be used to reduce the effects of blue light. In patients with Dry Eye, blue light filters (wrap around goggles) have been used to try to improve computer vision syndrome. Researchers showed reduced ocular complaint scores with all filter levels. Other studies have shown a reduction in critical flicker-fusion frequency and pain around/inside the eyes and less feeling of itchy eyes post-task. Blue-light filtering spectacle lenses can partially filter high-energy short-wavelength light without substantially degrading visual performance and sleep quality. These lenses may serve as a supplementary option for protecting the retina from potential blue-light hazard.

Blue light filters have been used *in vitro.* One such study looked at blue light irradiation of primary human cells of corneal surface origin. An assessment of viability 24 hours post blue light dose concluded that blue light injures ocular surface cells. Cells are protected from damage by a shade. Researchers recommended blue light protection to maintain ocular health, especially in high-risk populations, such as people with dry eye, contact lens users, the malnourished and the elderly. Indeed, implantation of blue light-filtering intra ocular lenses, which mimic the natural human lens have been used since the 1980s in cataract patients. Reviews of the available clinical studies on these intraocular filters suggests that they should be considered as a safe and relatively inexpensive preventive measure to reduce the potential risk for retinal phototoxicity and its associated potential risk for age-related macular degeneration (AMD) in pseudophakic eyes.

### Prolonged retention time

As indicated herein above, the eye surface is a unique tissue which creates a challenging environment in which to deliver therapeutic molecules in therapeutically effective amounts. The smoothness of the surface, the constant flow of tears, and regular sweeping of the eye lids reduce the amount of time available for absorption of therapeutic molecules to the corneal epithelium. This is challenging for hydrophilic molecules and even more problematic for lipophilic molecules, which must overcome their physico-chemical rejection from the polar environment of the eye's surface. Given that Vitamin D is an oily/lipophilic molecule, it has a very short retention time on the surface of the eye when administered topically thereto. This phenomenon is due to the natural aqueous environment of the eye's surface and due to the polarity of the corneal epithelium in combination with the tear flow and the eye lids sweeping effect. The resultant rapid removal of Vitamin D from the surface of the eye makes it very difficult to administer topical Vitamin D in effective amounts to the eye to provide therapy or nutrition. The surprising discovery that particular combinations of Vitamin D in phospholipid-comprising nano-emulsions exhibit longer retention times on the eye surface when applied topically to the eye and thereby facilitating enhanced absorption of Vitamin D by the corneal epithelium has solved the problem of how to administer therapeutically and/or nutritiously effective amounts of Vitamin D to the eye topically without causing irritation. These findings have led to the topical, ocular phospholipid comprising nano-emulsion formulations comprising Vitamin D and methods for using same.

### Concentration and ratios

In view of the above, specific formulations of vitamin D in a phospholipid nano-emulsion are envisioned, wherein the particle size of the emulsified particles is in a range having a diameter of 100-300 nanometers; the concentration of vitamin D is in the range of 0.02% and 2% weight/weight; and the phospholipids are in a specific range of ratios, typically 3:1 with the vitamin D content in the formulation. Vitamin D included in formulations described herein may be in a non-active, partially active, or active form.

In a particular embodiment, a phospholipid containing nano-emulsion comprising Vitamin D is as follows:
- An inert nano-emulsion (with no API) comprises 0.3% w/v of phospholipids;
- Phospholipids comprise at least one of egg and soy phospholipids;
- The concentration of phospholipids varies from 0.1% to 10% w/v;
- The Vitamin D concentration ranges from 0.001 to 1% w/v. In a particular embodiment the Vitamin D concentration is 0.01% w/v;
- The phospholipid containing nano-emulsion comprising Vitamin D is administered dropwise, wherein each drop is approximately 0.05 ml.

In a particular embodiment, formulations/compositions comprising Vitamin D in a phospholipid nano-emulsion described herein do not comprise a liquefied or compressed gas propellant. In other words, formulations/compositions comprising Vitamin D in a phospholipid nano-emulsion as described herein comprise 0% liquefied or compressed gas propellant. Accordingly, the present formulations/compositions comprising Vitamin D in a phospholipid nano-emulsion are not foam aerosols.

Formulations described herein are transparent and do not comprise strong surfactants that are used in foam aerosols. Such strong surfactants are contraindicated at least because they would irritate the eye.

As understood in the art and defined in, for example, the US Pharmacopeia, a propellant supplies the necessary pressure within an aerosol system to expel material from a container and, in combination with other components, to convert the material in which is included into a desired physical form. Propellants are broadly classified as liquefied or compressed gases having vapor pressures generally exceeding atmospheric pressure.

As understood in the art and defined in, for example, the US Pharmacopeia, a foam aerosol is an emulsion containing one or more active ingredients, surfactants, aqueous or nonaqueous liquids, and a propellant/s.

### Particle Size

As described herein, emulsified particles in the phospholipid nano-emulsion have a particle size ranging from 100-300 nanometers in diameter. In particular embodiments, emulsified particles in the phospholipid nano-emulsion have a particle size ranging from 100-150, 100-200, 100-250, 100-300, 150-200, 150-250, 150-300, 200-250, 200-300, or 250-300 nanometers (nm) in diameter. In a particular embodiment, emulsified particles in the phospholipid nano-emulsion have a particle size of about 200 nm in diameter. In a particular embodiment, emulsified particles in the phospholipid nano-emulsion comprise a population of emulsified particles having an average particle size of about 200 nm in diameter. In a further particular embodiment, emulsified particles in the phospholipid nano-emulsion comprise a population of emulsified particles, wherein equal to or greater than 50%, 55%, 60%, 65%, 70%, 75%, 80%, 85%, 90%, 95%, 96%, 97%, 98%, or 99% of the population has an average particle size of about 200 nm in diameter.

### Vitamin D

Vitamin D plays an essential role in the human body and is important in maintaining normal function in a variety of organ systems. Our knowledge regarding the impact of Vitamin D deficiencies is growing exponentially and health professionals are addressing the newly appreciated needs by prescribing regular supplementation of Vitamin D. Organ systems and functions thereof adversely impacted by Vitamin D deficiencies include, without limitation: bone mineralization, muscle weakness, the immune system, and the cardiovascular system.

Scientists and medical practitioners have recently focused their attention on the importance of Vitamin D to the ocular system. Vitamin D has been shown to contribute enormously to the vitality and the health of the eyes with a significant involvement at both the anterior (front) and at the posterior (back) of the eye.

Corneal cells, for example, can convert Vitamin D to its active metabolite 1,25D3 (Vitamin D3), in a manner resembling the conversion process that occurs in the skin. Moreover, corneal epithelial cells have also been shown to produce Vitamin D de novo, when exposed to UVB. These basic abilities reveal a biological significance of Vitamin D in the ocular system and corroborate number of basic science and clinical studies that implicated Vitamin D and the ocular system and functions thereof. Further evidence of a link between Vitamin D and the ocular system is found in the identification of a positive correlation between myopia and serum Vitamin D levels in a large population of human subjects examined. Additional studies have confirmed these findings and affirmed that myopic participants examined had lower Vitamin D levels. Optical coherence tomography (CT) to measure pathological changes preceding disease has also revealed that Vitamin D insufficiency is associated with reduced macular thickness. Additional studies have shown that age-related macular degeneration (AMD) patients with subretinal fibrosis had significantly lower serum Vitamin D levels than patients without fibrosis.

The ability of Vitamin D to inhibit neovascularization has led researchers to examine its involvement in diabetic retinopathy development. Epidemiological studies have reported that serum Vitamin D concentration is inversely related to the severity of retinopathy in diabetic patients. Genetic variations in Vitamin D metabolism have also been linked to uveitis, an inflammation of the middle layer of the eyeball known as the uvea.

Other studies have provided evidence that Vitamin D supplementation could be beneficial not only during an active inflammatory condition but also for prevention of such conditions by maintaining normal eye health and function.

In a particular embodiment, a pharmaceutical composition described herein is administered in the form of eye drops or eye salves. In a more particular embodiment, a pharmaceutical composition described herein is administered in the form of eye drops or eye salves that further comprise ophthalmologically acceptable carriers. As described herein, the composition is in the form of a nano-emulsion.

For some indications, a pharmaceutical composition described herein is a hypotonic composition. **In** a particular embodiment thereof, a hypotonic pharmaceutical preparation is used wherein a disorder of the eye is associated with a high concentration of salts on the surface of the eye. **In** another embodiment, physico-chemical constraints on a specific pharmaceutical formulation call for a hypotonic preparation. Typically, however, formulations to be administered to the eye are isotonic ocular preparations so as to minimize irritation of eyes to which they have been applied. The unique composition of phospholipid nano-emulsion formulations described herein, however, provides a hypotonic ocular preparation that does not irritate the eye. This feature of the nano-emulsion formulations described herein provides patients in need thereof with new options for therapeutic intervention which were impossible or very inconvenient in advance of the present discoveries.

For some indications, a pharmaceutical composition described herein is a hyperosmotic composition. In a particular embodiment thereof, a hypotonic pharmaceutical preparation is used wherein a disorder of the eye to be treated is associated with diminished liquid clearance from the eye and resultant water retention, which eventually leads to rupture of the eye membranes. In such cases, compositions described herein are presented in a hyperosmotic formulation which serves to draw excess liquid from the eye. Such hyperosmotic formulations may be formed, for example, by the addition of salts (e.g., NaCl) to the composition.

Furthermore, some physico-chemical properties of pharmaceutically active ingredients call for the use of hypertonic formulations in ocular preparations. The high osmotic pressure of such ocular pharmaceuticals, however, is irritating to the eye and thus, limits the use of otherwise effective therapeutic agents for eye disorders. The pharmaceutical preparations described herein diminish the irritating effects of hyperosmotic preparations and in so doing, provide new opportunities for developing ocular pharmaceuticals comprising therapeutic agents which could not previously be formulated as ocular treatments due to their hypertonicity. Pharmaceutical preparations described herein may also provide opportunities to reformulate existing hypertonic preparations to generate less irritation ocular treatments.

In yet another aspect, a formulation or composition for storing and maintaining isolated corneas (e.g., in an eye repository) is envisioned. In order to maintain the viability of epithelial cells as well as the exposed endothelium of the eye, it is necessary to enable the penetration of lipophilic nutrients to the corneal cells. The nano-emulsion formulations described herein may be used as a storage medium for isolated corneas that optimizes the preserved tissue's shelf life and quality.

### Transparency properties of Vitamin D nano-emulsions

The present inventors have demonstrated that at visible light wave lengths (400-700 nm), phospholipid containing nano-emulsions comprising Vitamin D do not scatter light and therefore, would not obstruct the eyesight of a subject (e.g., a human patient or animal subject) treated with same. These findings are presented in, for example, [0034]. As shown in [0034], high resolution images can be seen through the layer of the nano-emulsion in the visible light spectral range (white light lamp). The thin layer mimics the actual thickness of the tear layer on the cornea. These findings illustrate a significant property of the present phospholipid containing nano-emulsions comprising Vitamin D described herein.

### Additional physical/structural properties of Vitamin D nano-emulsions

The present inventors have further characterized the phospholipid containing nano-emulsions comprising Vitamin D described herein by analyzing the size of the emulsified particles and the diameter of any air bubbles in the nano-emulsions. Their analyses have shown that very few air bubbles are found in the present nano-emulsions. As shown in [0032], which is a photograph of a microscope slide on which a phospholipid containing nano-emulsion comprising Vitamin D has been placed, only 5 air bubbles were found in an area of 3x5 m² of nano-emulsion formulation. The diameter of the bubbles is around 90 microns. The present inventors also determined that the size of the emulsified particles in phospholipid containing nano-emulsions comprising vitamin is around 200 nanometers.

In another experiment, the present inventors microscopically examined a phospholipid containing nano-emulsion comprising Vitamin D and determined that no bubbles could be detected in slide area of 7X5 mm² wherein the nano-emulsion had been spread evenly between two glasses. See [0033].

The present inventors also examined the clarity of the phospholipid containing nanoemulsion comprising Vitamin D and found that resolution is well preserved through a thin layer of the nano-emulsion. See [0035]. This determination was made relative to a reference resolution of 20 lines of 60 nanometer in width, over a 1200 nanometer line, as observed using 40X magnitude. See [0034].

Results presented in, for example, [0032] to [0035] demonstrate that the phospholipid containing nano-emulsions comprising Vitamin D described herein are transparent and do not impair resolution. Accordingly, the phospholipid containing nano-emulsions comprising Vitamin D described herein possess properties that will preserve visual acuity of a subject to whom the nano-emulsions have been administered topically to the eyes. Results presented herein also demonstrate that the present nano-emulsions are not foams.

### Vitamin D Cell Penetration Comparison Studies

Vitamin D absorption can be determined using a human corneal limbal epithelial cell line as a model system for corneal epithelial cell absorption. In a particular embodiment, Vitamin D absorption from a phospholipid-containing nano-emulsion comprising Vitamin D can be compared to absorption from an oily preparation comprising Vitamin D.

Briefly, cells are incubated in either an oily medium/preparation or in a phospholipid-containing nano-emulsion medium, wherein each comprises 10 µM 7-dehydrocholesterol for 24 hours. Cells are then exposed to UV-B (302 nm). Cells are replenished with fresh medium immediately prior to UV-B exposure. Medium is not changed after the initial UV-B exposure to allow for accumulation of synthesized Vitamin D metabolites. Cells are exposed to UV-B twice a day, at a total dose of 10 or 20 mJ/cm²/day in divided doses, administered 6 hours apart for three days. The medium is collected 1 hour after the final UV-B exposure and stored at -80°C until mass spectroscopy analysis is performed to analyze Vitamin D metabolites generated.

Alternatively, or in addition, cells may be incubated in an oily medium/preparation or in a phospholipid-containing nano-emulsion medium, wherein each comprises 10 µM 7-dehydrocholesterol for 24 hours. Culture medium can then be replenished with fresh medium and the Vitamin D content of the two differentially treated cell cultures can be compared using mass spectroscopy analysis for Vitamin D.

### Protective effects conferred by Vitamin D nano-emulsions

Spectrophotometric tests, i.e. scanning through the light wavelength (energy) and evaluation of absorbance at each certain energy were used to assess protective effects of Vitamin D nano-emulsions described herein. When administered to the eye, Vitamin D nano-emulsions described herein provide the effect of "sunglasses", without putting on glasses. Normally people use sunglasses when exposed to daylight to block exposure to various frequencies of light. However, when reading from screens or working in artificial light environments, people do not typically use protective glasses. Vitamin D nano-emulsions described herein provide enhanced UV protection throughout the spectral range [UV C: 100-280nm, UV B: 280-315nm, UV A: 315-400nm, Blue-light: 400-495nm]. Protection in the blue light range is particularly important with respect to protection from light emissions from screens and artificial light environments. Accordingly, protection is conferred by administration of Vitamin D nano-emulsions described herein in the full UV + Blue light range (100-495nm), including each of the following spectral ranges: UV A, B, and C and the Blue Light range.

[0036] to [0042] presents results with the NanoEm (Lipimix matrix) and the Vit-D NanoEm [Lipimix loaded with the vit-D (vitamin-D formulation)] that was diluted as indicated. The particles (spheres) sizes and the ratios (PL:vit-D) are, therefore, unchanged and only the Vitamin D concentration (wt/wt) is changing.

Each of the indicated formulations were tested for absorption (on the same tool, continuous runs) over a wide energy range (wavelengths), covering the UV A, B, C and the blue light spectra. Surprising and unexpected effects are shown in, for example, [0041], which reveals that incorporation of the vit-D into the spheres drastically changes the absorption characteristics of the matrix. The absorption increase observed far exceeds a simple accumulation of effects (as in summing the vit-D in water + the Lipimix matrix absorption values). Surprising and unexpected effects are shown in, for example, [0040], which reveals that repeatedly diluting the vit-D nano-emulsion doesn't cause a constant decrease in absorption. This finding is surprising at least because one would expect that reducing the spheres density would yield a parallel decrease in absorption. This is particularly apparent in the Lipimix absorption observed between the 2^{nd} and the 3^{rd} dilution of the vit-D formulation.

Further to the above, sun protection factor (SPF) is routinely measured by spectrophotometry. It is a well-accepted method and commonly used in the literature to evaluate, for example, UV light damage to skin.

### UVB measurement of Lipitear and Lipicare SPF eye-drops

Another assay described herein uses a device (Digital UV Meter) that integrates over the whole UVB range to see how much energy is reaching the detector, which serves as an equivalent to the surface of the eye. Briefly: a thin layer of nano-emulsion can be formed between 2 transparent polymer stripes, mimicking the thickness of the layer on the eye. In daytime (1000 and 1430 hour):
- "UVB Energy in mW/cm² Baseline" - Present inventors measured the total energy of the UVB spectral range using an exposed detector. This represents the amount of potentially hazardous radiation that can reach the eye in this specific spectral range.
- "Polymer Baseline" - Present inventors repeated the test with 2 blank polymer frames to see the effect of those on the test (no liquid in between).
- "UVB Energy in mW/cm² - Inert Nano-emulsion" - Present inventors added a nanoemulsion with similar physical characteristics but with no vit-D (the empty matrix alone) and demonstrated no reduction was obtained in the total amount of radiation reaching the eye.
- "UVB Energy in mW/cm² - Vitamin D Nano-emulsion" - Present inventors replaced the empty matrix with a vit-D containing nano-emulsion (a loaded matrix) and demonstrated 50-70% reduction in the amount of UVB light reaching the eye.

### Winter time Israel

Altitude 0-50m OSL
Method: 0.3 ml of liquid between two layers of transparent poly propylene 45micron sheet, 2x3 cm2

| Time on day | UVB Energy in mW/cm2 Base line | Polymer baseline | UVB Energy in mW/cm2 Inert Nanoemulsion | UVB Energy in mW/cm2 Vitamin D Nanoemulsion | Vitamin D Nano emulsion UV % reduction |
|---|---|---|---|---|---|
| 10:00 | 0.14 | 0.13 | 0.13 | 0.04 | 71.4% |
| 14:30 | 0.05 | 0.05 | 0.04 | 0.025 | 50% |

Accordingly, the vit-D containing nano-emulsion conferred an absorption property in this assay, thereby corroborating other results presented herein.

### OCULAR SAFETY STUDY OF LIPITEAR^{™} IN RABBITS

### OBJECTIVE

The purpose of this study was to test the safety of three Test Items compared to two comparators, formulated in LIPITEAR^{™}, following topical administration into rabbits' eyes.

Clinical observation three times during the first 24 hours after dosing with special attention during the first four hours and twice daily thereafter for three days total. Changes in skin and fur, specifically around the eyes and mucous membrane.

Ophthalmic evaluation (By Draize Scale, both eyes); baseline, 1h post administration and once daily at 24h, 48h, 72h, 96h and 120h post first administration by lamp for all study groups (last evaluation for both eyes).
Morbidity and mortality - twice a day (once daily over the weekend).
Body weight measurements - before study initiation

### Justification for the selection of test system

The NZW rabbits strain was used. Healthy adult animals (2.2-2.7kg at study initiation) of this commonly used laboratory strain were employed. The rabbits were the chosen species for the study, and historically have been used for safety ocular studies.

### Rationale for test group size

A total of 15 rabbits were utilized and divided into five groups of three rabbits each: one Vitamin D eye drops group (Vitamin D-Lipitear formulation; Test Item 1); Alternate Compound A eye drops group (Compound A -Lipitear formulation; Test Item 2); one reference: Lipitear (existing product) group; Alternate Compound B eye drops group (Compound B-Lipitear formulation; Test Item 3) and one Reference: comparator product group. The concentration of Vitamin D in the Vitamin D-Lipitear formulation is 100mg Vitamin D in 100ml of final emulsion. The number of the groups and the total number of animals is the minimum number of animals per group sufficient to obtain indicative/significant information.

### EXPERIMENTAL MODEL

Animals
Species / Strain
Rabbit / NZW
Gender / Number / Age
Males / 15 / 2.2-2.7 kg at study initiation
Source
Envigo RMS (Israel) Ltd
Body weight

The animals' average (±SD) BW at study initiation was 2.4 ± 0.2 kg. The minimum and maximum weight of the animals was within a range of ±20% of the group's mean weight.

### Acclimation period:

Animals were acclimated for seven days. Animals remained at Pharmaseed until study initiation for additional 20 days until they reached the required body weight.

### Identification:

Animals were identified by the breeder by dye ear numbers. This number also appeared on a cage card, visible on the front of each cage. The cage card additionally contained the study number and relevant details as to the treatment group.

### Animal Management

### Housing

Animal handling was performed according to guidelines of the National Institute of Health (NIH) and the Association for Assessment and Accreditation of Laboratory Animal Care (AAALAC). Rabbits were housed individually in plastic cages mounted in batteries. The cages measure is 60 (L) x 60 (W) x 40 (H) cm. Cages were fitted with perforated plastic floors over under-trays. Stairs and PVC cylinders were supplied for recreational enrichment.

### Diet

Commercial rabbit diet (Teklad Doe Rabbit Diet cat#: 7078S) was provided and the animals had free access to the food and to acidified drinking water (pH between 2.5 and 3.5) obtained from the municipality supply and treated according to Pharmaseed's SOP No. 214 "Water System".

### Contaminants

The feed arrived from the vendor (Envigo) with a certificate of analysis. The water was treated as above.

### Environment conditions

Animals were housed under standard laboratory conditions, air conditioned and filtered (HEPA F6/6) with adequate fresh air supply (Minimum 15 air changes/hour). Animals were kept in a climate controlled environment. Temperatures range was 18-240C and relative humidity range 30-70% with a 12 hours light and 12 hours dark cycle (6AM/6PM).

### Randomization

Rabbits were stratified into one of the five groups according to their body weight.

### EXPERIMENTAL DESIGN AND CONDITIONS

### Pain reducing methods

The procedure did not cause pain to the animals during the study and no animal showed severe clinical symptoms during the study. Therefore, no analgesics were needed.

### Humane Endpoints

No animal from any treated groups was euthanized during this study on humane ground.

### Duration of the experimental period

First dosing day was considered Day 1 and termination day of most animals was Day 6. Due to reaction signs observed in one animal (#8168), an additional examination was performed on Day 8 and the minor redness was still observed. Since this was an animal treated by a comparator, and none of the other Test Items had shown any eye reactions on Days 5 and 6, no further examination was performed and the study was terminated on Day 8.

### Study design

The study was conducted in one cycle, according to Table 1 and Table 2.

### Administration of the Test Items

The test substance was placed in the conjunctival sac of one eye of each animal after gently pulling the lower lid away from the eyeball. The lids were then gently held together for about one second in order to prevent loss of the material. The other eye, which remains untreated, served as a control. Each Test and Reference Item was administrated to three animals per group, according to Table 1. Study time line was according to Table 2. Dosing regimens were according to Table 3.

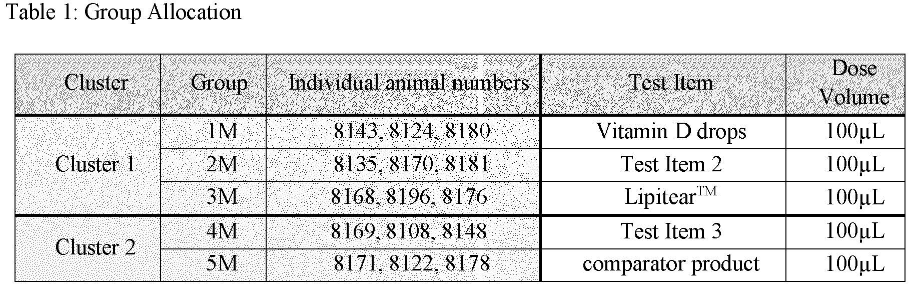

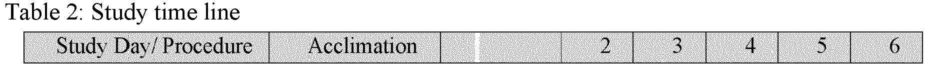

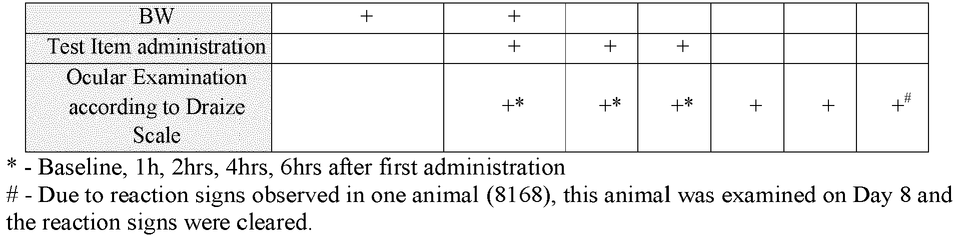

**Table 3: Dosing Regimens***

| Day | Cluster 1 | Cluster 2 |
|---|---|---|
| 1 | 1^{st} administration and after no adverse events within an hour, three more applications, 1 hour apart | 1^{st} administration and after no adverse events within an hour, two more applications, 3 hour apart |
| 2 | Six applications, 1.5 hours apart | Three applications, 3 hours apart |
| 3 | Six applications, 1.5 hours apart | Three applications, 3 hours apart |

| | | |
|---|---|---|
| * The dosing regimen was exaggerated, but related to the expected clinical use | | |

### Tests and evaluations

### Morbidity and mortality observation

Morbidity and mortality check was performed twice a day (once daily over the weekend).

### Body weight

Body weight was recorded upon arrival and before study initiation, according to Pharmaseed's SOP No. 010 "Weighing Laboratory Animals".

### Clinical observations

Clinical observations were performed periodically during the first 24 h, with special attention during the first 4 h, and twice daily thereafter, for a total of three days. Changes in skin and fur, eyes and mucous membranes, in respiratory, circulatory, autonomic and central nervous systems, somatomotor activity and behavior pattern as tremors, convulsions, salivation, diarrhea, lethargy, sleep and coma. Special attention was directed to the eyes. Observations were documented on a specifically designed form.

### Ocular examination

The grading of ocular reaction was performed before Test Item administration (baseline), and at approximately (1±0.1)h, (2±0.1)h, (4±0.1)h, (6±0.1)h, (24±0.2)h, (48±0.2)h, (72±2)h, (96±2)h and (120±2)h following the first Test Item administration (according to Table 4 as required in ISO 10993-10:2010 section B.2.7). On days 2 and 3 an additional examinations were performed approximately (1±0.1)h, (2±0.1)h, (4±0.1)h, (6±0.1)h after the first administration.

Extended observation until 168 hours was necessary for one animal, in order to determine the reversal of the lesion.

**Table 4: Ocular irritation scoring - Draize score**

| 1. Cornea | Numerical Grading |
|---|---|
| Degree of opacity (most dense area) | |
| No opacity | 0 |
| Scattered or diffuse areas, details of iris clearly visible | 1^{a} |
| Easily discernible translucent areas, details of iris slightly obscured | 2^{a} |
| Opalescent areas, no details of iris visible, size of pupil barely discernible | 3^{a} |
| Opaque, detail of iris not visible | 4^{a} |
| Area of cornea involved | |
| One-quarter (or less), not zero | 0 |
| Greater than one-quarter, but less than half | 1 |
| Greater than half, but less than three-quarters | 2 |
| Greater than three-quarters, up to whole area | 3 |

| 2. Iris | |
|---|---|
| Normal | 0 |
| Folds above normal, congestion swelling, circumcorneal injection (any or all or combination of these), iris still reacting to light (sluggish reaction is positive) | 1^{a} |
| No reaction to light, haemorrhage, gross destruction (any or all of these) | 2^{a} |

| 3. Conjunctivae | |
|---|---|
| Redness (refers to palpebral and bulbar conjunctiva excluding cornea and iris) | |
| Vessels normal | 0 |
| Vessels definitely injected above normal | 1 |
| More diffuse, deeper crimson red, individual vessels not easily discernible | 2^{a} |
| Diffuse beefy red | 3^{a} |
| Chemosis | |
| No swelling | 1 |
| Any swelling above normal (include nictitating membrane) | 1^{a} |
| Obvious swelling with partial eversion of lids | 2^{a} |
| Swelling with lids about half-closed 3 | 3^{a} |
| Swelling with lids about half-closed to completely closed | 4^{a} |
| Discharge | |
| No discharge | 0 |
| Any amount different from normal (does not include small amounts observed in inner canthus of normal animals) | 1 |
| Discharge with moistening of the lids and hairs just adjacent to lids | 2 |
| Discharge with moistening of lids and hairs, and considerable area around the eye | 3 |
| ^{a} - Positive result | |

According to the ISO guideline, a test material is considered an eye irritant if more than half of the animals in the test group exhibit a positive result (footnoted grades given in Table 4) at any stage of the observation.

### Study Termination

According to ocular lesions results, 120 hours or 168 hours (animal #8168) post first Test Item administration, the study was concluded. At study termination animals were sent to rehabilitation according to Pharmaseed's SOP No. 043 "Releasing rabbits and guinea pigs for rehabilitation".

### RESULTS

### Morbidity and mortality observation

No animal from any of the groups died or was observed with signs of morbidity during the study.

### Body weight (BW) measurements

All animals were in the required range at study initiation. Body Weights upon reception and at study initiation are presented in Table 5.

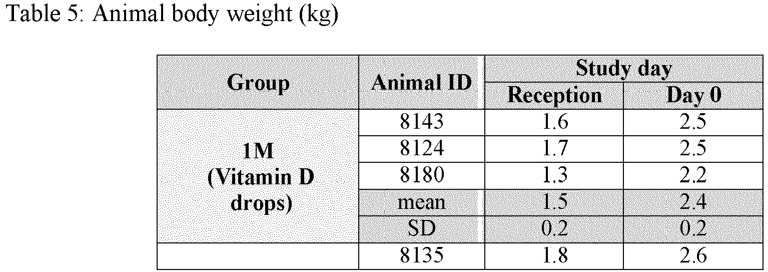

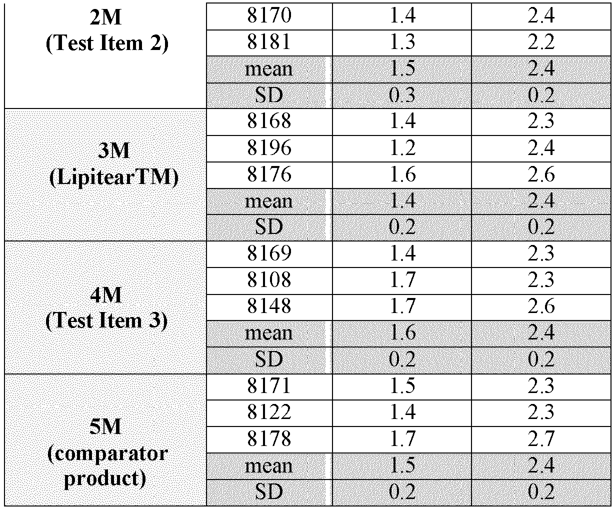

### Clinical signs

No abnormal clinical signs were observed in any of the animals during the study period. Individual clinical sign observations are presented in Table 6.

**Table 6: Individual clinical sign observations**

| **Group** | **Animal ID** | **Day 1** | | **Day2** | | **Day 3** | |
|---|---|---|---|---|---|---|---|
| | | **Predose** | **4 hrs** | **Predose** | **4 hrs** | **Predose** | **4 hrs** |
| **1M (Vitamin D drops)** | 8143 | NAD | NAD | NAD | NAD | NAD | NAD |
| | 8124 | NAD | NAD | NAD | NAD | NAD | NAD |
| | 8180 | NAD | NAD | NAD | NAD | NAD | NAD |
| **2M (Test Item 2)** | 8135 | NAD | NAD | NAD | NAD | NAD | NAD |
| | 8170 | NAD | NAD | NAD | NAD | NAD | NAD |
| | 8181 | NAD | NAD | NAD | NAD | NAD | NAD |
| **3M (Lipitear^{™})** | 8168 | NAD | NAD | NAD | NAD | NAD | NAD |
| | 8196 | NAD | NAD | NAD | NAD | NAD | NAD |
| | 8176 | NAD | NAD | NAD | NAD | NAD | NAD |
| **4M (Test Item 3)** | 8169 | NAD | NAD | NAD | NAD | NAD | NAD |
| | 8108 | NAD | NAD | NAD | NAD | NAD | NAD |
| | 8148 | NAD | NAD | NAD | NAD | NAD | NAD |
| **5M (comparator product)** | 8171 | NAD | NAD | NAD | NAD | NAD | NAD |
| | 8122 | NAD | NAD | NAD | NAD | NAD | NAD |
| | 8178 | NAD | NAD | NAD | NAD | NAD | NAD |

| | | | | | | | |
|---|---|---|---|---|---|---|---|
| NAD - No abnormalities detected | | | | | | | |

### Ocular examination using Draize test

There were only minor signs of eye reactions or abnormalities observed that could be related to the Test Items or Comparators. Minor conjunctiva redness was the only sign of eye reaction observed. The maximal numerical grading of conjunctivae redness observed during this study was "1". According to the ISO guideline, a numerical grading of "0" or "1" for conjunctivae redness is not considered as a positive grade. None of the animals exhibited a positive result indicating an adverse effect and therefore none of the Test Items administrated in this study should be considered as an eye irritant.

This minor redness was observed in animal No. 8168 from Group No. 3 (Lipitear) on Day 6. An additional examination was performed on Day 8 and the minor redness was still observed. Since Lipitear is a comparator, and none of the other Test Items had shown any eye reaction on Days 5 and 6, no further examination was performed and the study was terminated on Day 8.

### DISCUSSION AND CONCLUSION

No animal from any of the groups died or was observed with signs of morbidity during the study. No abnormal clinical signs were observed in any of the animals during the study period. No long-term signs of eye irritation or abnormalities were observed that could be related to the Test Items or Comparators. None of the animals exhibited an adverse effect in the ocular examination and all the minor transient reactions and abnormalities observed were considered related to the administration, were observed in both the study and control groups and are considered acceptable in this kind of procedure.

Based on the above findings and under the conditions of this study, it is concluded that a three-day regimen comprising repeated administration of the Tested Items onto a rabbit eye was well tolerated and did not cause any significant adverse clinical effects compared to commercial comparators treatment. None of the Test Items administrated in this study should be considered an eye irritant.

Variations, modifications and alterations to embodiments of the present disclosure described above will make themselves apparent to those skilled in the art. All such variations, modifications, alterations and the like are intended to fall within the spirit and scope of the present disclosure, limited solely by the appended claims.

While several embodiments of the present disclosure have been described, it is understood that these embodiments are illustrative only, and not restrictive, and that many modifications may become apparent to those of ordinary skill in the art. For example, all dimensions discussed herein are provided as examples only, and are intended to be illustrative and not restrictive.

Any feature or element that is positively identified in this description may also be specifically excluded as a feature or element of an embodiment of the present as defined in the claims.

The disclosure described herein may be practiced in the absence of any element or elements, limitation or limitations, which is not specifically disclosed herein. Thus, for example, in each instance herein, any of the terms "comprising," "consisting essentially of and "consisting of" may be replaced with either of the other two terms. The terms and expressions which have been employed are used as terms of description and not of limitation, and there is no intention in the use of such terms and expressions of excluding any equivalents of the features shown and described or portions thereof, but it is recognized that various modifications are possible within the scope of the disclosure.

### LIPITEAR^{™} Absorption Test Summary

An exemplary nano-emulsion, Lipimix nano-emulsion (LIPITEAR^{™}), was selected to be assessed as a delivery mechanism for active ingredients to the surface of the eye. The results presented in this example are not, however, limited to use of LIPITEAR^{™}.

The phospholipid nano-emulsions comprising Vitamin D create a surprising physical ability to filter electro-magnetic radiation at various wave lengths. The Vitamin D phospholipid nano-emulsion, once placed over the eye was found to filter Blue light and UV radiation. This property is surprising not just qualitatively, but also quantitively, since the thickness of the Vitamin D Nano-emulsion on the surface of the eye corresponds to the thickness of the tear film and yet ,the ability of the Vitamin D nano-emulsion to filter electro-magnetic radiation is significant.

### UV Radiation and the eye

The cornea, like the skin, is highly exposed to ultraviolet radiation (UVR). Epidemiological data indicate that UVR is a contributing factor for a multitude of diseases of the cornea including pterygium, photokeratitis, climatic droplet keratopathy and ocular surface squamous neoplasia (OSSN). UVR is a well-known genotoxic agent, and its effects have been well characterized in organs such as the skin. Several effects of UVR on the cornea are identified, such as the UVR signature C → T and CC → TT transversions identified by sequencing and increased proliferative and shedding rates in response to UVR exposure. A single low-dose exposure of UVR to the cornea is sufficient to elicit genetic, molecular and cellular changes, supporting the consideration of using protective measures on the cornea.

The Vitamin D Nano-emulsion described herein filters UVR and Blue light in an extent that may provide a protecting effect to the eye.

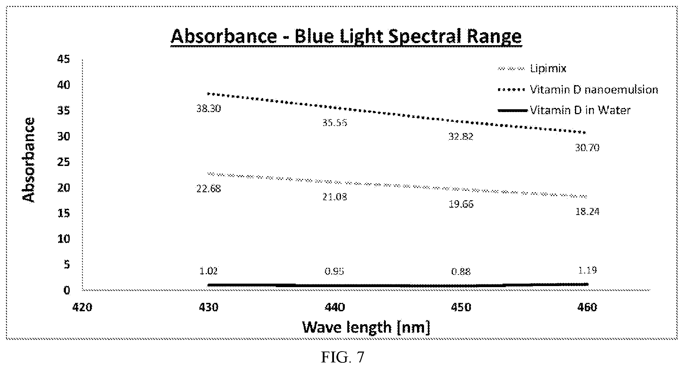

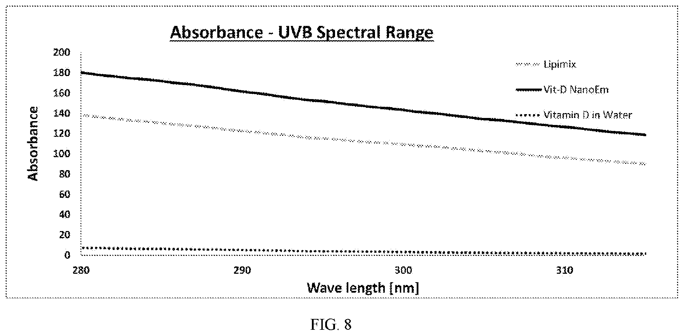

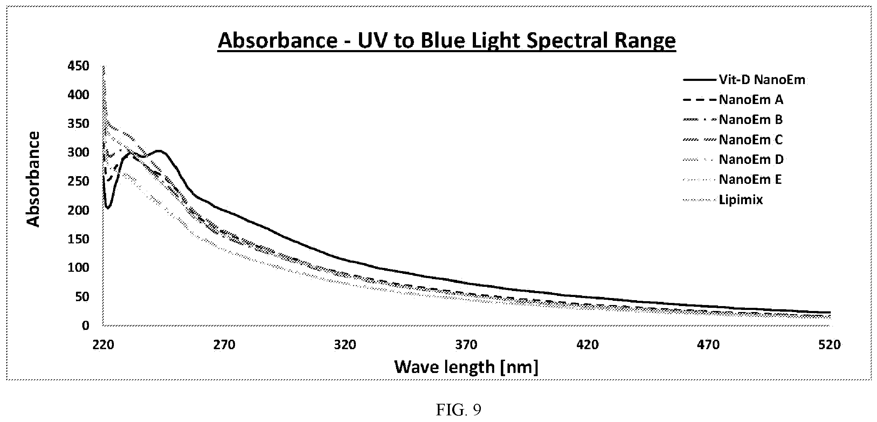

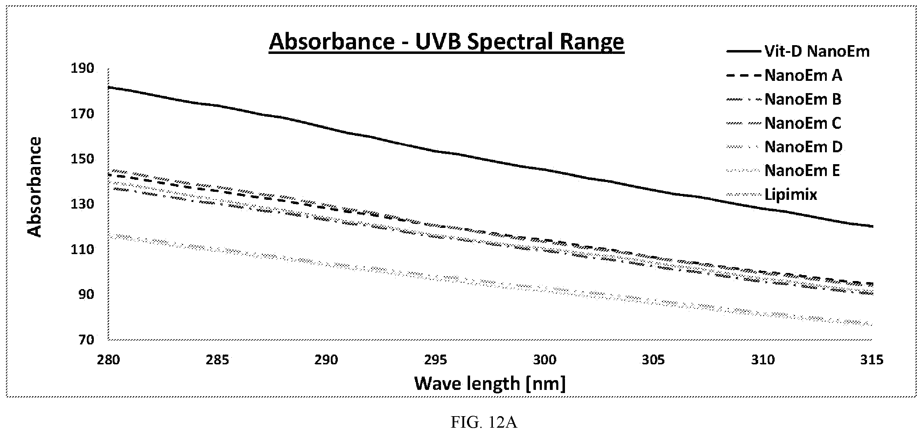

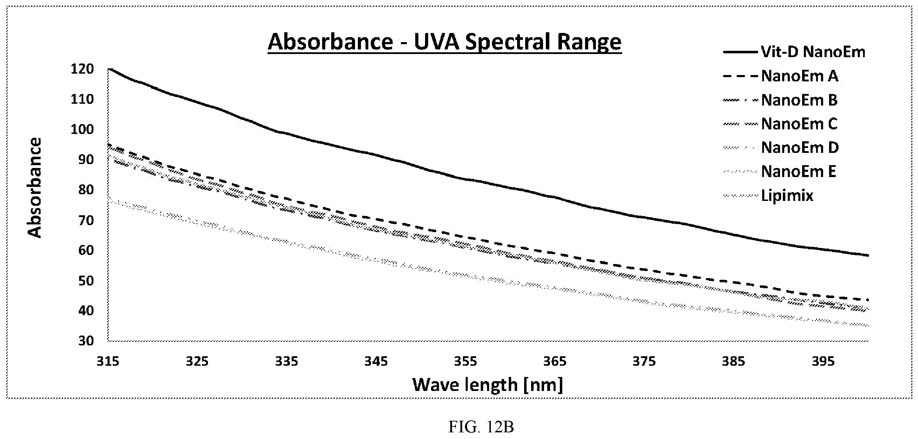

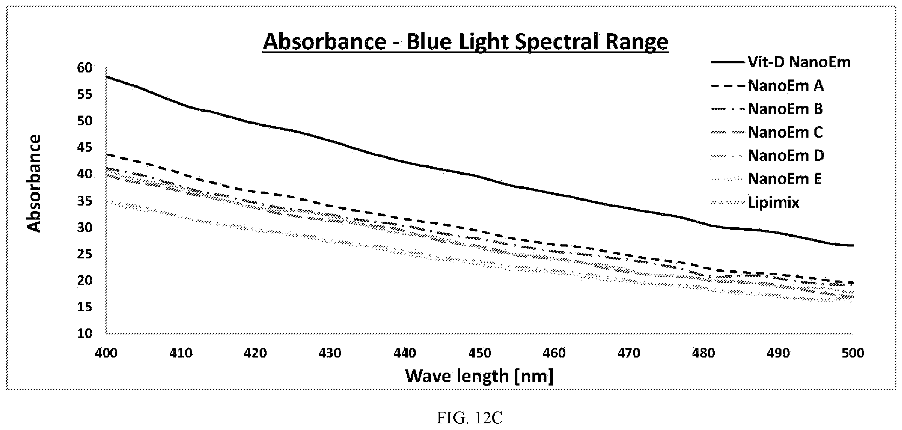

Compositions comprising different concentrations of Vitamin-D (Vit-D) were assessed for their activity with respect to the eye. The following describes absorbance tests conducted on compositions comprising Vit-D formulated into LIPITEAR^{™}. The compositions are intended to be used as eye drops and at a target formulation comprise 100mg of Vit-D in 100ml of nanoemulsion or 4% of the lipid fraction comprising the Nano-emulsion. Characteristics of Vit-D concentration dependent absorbance were assessed and are summarized below. See also FIGS. 7-13. Given that the compositions are diluted by tears when administered to an eye, the effect of aqueous dilution was also evaluated (See Table 8 below and [0040]).

### Experimental

A Denovix DS-11 FX spectrophotometer unit was used for all tests. All formulations were assessed one after the other in a single session. The wavelength range of 220-520nm was captured, specifically covering the blue-light range (400-495nm) and the UV tail (□<400nm).

Two sets of formulations were tested:
1. A set of 6 nano emulsions was prepared, starting with two source formulations of LIPITEAR^{™} and Vit-D NanoEm and by serially diluting the latter 5 times in nanoemulsion as follows:

**Table 7: the set of formulations measured.**

| | Formulation Name | Vit-D / Lipimix [mg/100ml] |
|---|---|---|
| 1 | Vit-D NanoEm | 100.0 |
| 2 | NanoEm A | 50.0 |
| 3 | NanoEm B | 25.0 |
| 4 | NanoEm C | 12.5 |
| 5 | NanoEm D | 6.3 |
| 6 | NanoEm E | 3.1 |
| 7 | Lipimix (LIPITEAR^{™}) | 0 |

2. A set of similar formulations diluted at a 1:4 ratio named Vit-D NanoEm 0.2, Vit-D NanoEm 0.2 A etc.

| | Formulation Name | Vit-D / Lipimix [mg/100ml] |
|---|---|---|
| 1 | Vit-D NanoEm 0.2 | 20.0 |
| 2 | NanoEm 0.2A | 10.0 |
| 3 | NanoEm 0.2B | 5.0 |
| 4 | NanoEm 0.2C | 2.5 |
| 5 | NanoEm 0.2D | ~1.3 |
| 6 | NanoEm 0.2E | ~0.6 |
| 7 | Lipimix (LIPITEAR^{™}) 0.2 | 0 |
| ***Table 8:*** the set of formulations measured. | | |

The preparation was made of a single Vit-D NanoEm formulation by dilution only.

**Table 9: embodiments of pharmaceutical compositions described herein.**

| **Ingredient** | **Units** | **Specific Embodiment** | **Range of Embodiments** |
|---|---|---|---|
| Phospholipids | % wt/vol | 0.3 | 0.1-5 |
| Vitamin D | % wt/vol | 0.1 | 0.02-2 |
| Vitamin D in oil phase | % wt/vol | 4 | 0.1-20 |
| Wave length | nm | 280-315, 415-490 | 220-520 |
| All % are weight in volume | | | |

### Results

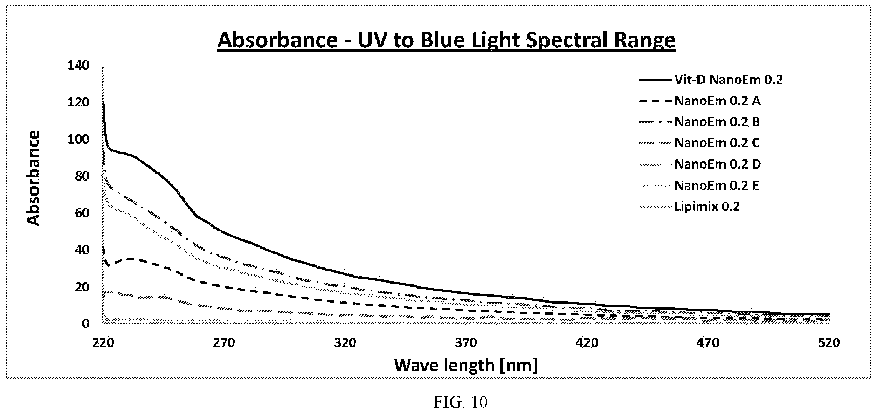

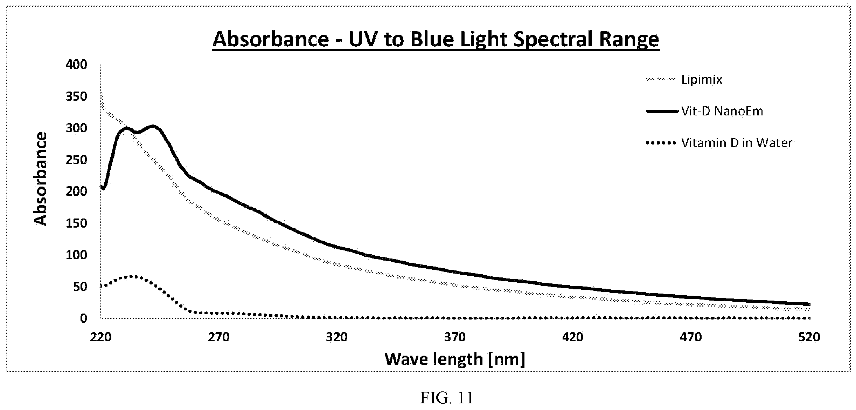

FIGS. 10 and 11 present the absorbance properties of the 2 formulation sets.

Figure 10 depicts the surprising properties of Vit-D in the compositions tested in the 220-520 nm range. The superiority absorption properties of Vit-D NanoEm (compositions comprising Vit-D as presented in Table 7) are well demonstrated at wavelengths exceeding 230 nm.

Figure 11 depicts the surprising properties of Vit-D NanoEm (compositions comprising Vit-D as presented in Table 8) in the 220-520 nm range, which properties are preserved and enhanced when diluted five-fold with water. A five-fold dilution was chosen to simulate the expected dilution when a composition as described herein is administered to an eye and is diluted by tears on the surface of the eye.

The absorption spectra at 238nm, for example, stabilizes in accordance with the Vit-D concentration and the absorption line maintain their relative arrangement all the way to the end of the measured range with minimal to no crossing of lines. See 0. An optimally aligned image can be seen for the water diluted formulations set throughout the spectrum range. See FIG. 11.

As depicted in 0, the absorption properties of the Vit-D composition can be divided into 3 efficacy groups, with the Vit-D NanoEm superior to all starting at 238nm, a group of fairly equivalent absorption of the Lipimix and Nano-emulsions A,B&C, and low absorption for Nano-emulsions D&E.

As depicted in [0040], the absorption properties of the Vit-D compositions when diluted five-fold (Table 8 concentrations) are generally consistent with those of the Vit-D compositions of Table 7. More particularly, the Vit-D NanoEm 0.2 is superior to all, followed by the Vit-D NanoEm 0.2B, Lipimix, Vit-D NanoEm 0.2A, Vit-D NanoEm 0.2C, Vit-D NanoEm 0.2E, and Vit-D NanoEm 0.2D. Vit-D NanoEm 0.2D and Vit-D NanoEm 0.2E have negligible absorbance.

For reference, a separate absorption spectrum of Vit-D in a distilled water solution compared with Lipimix and Vit-D NanoEm was recorded and is presented in [0041]. [0041] depicts a comparison of the absorption spectra of Lipimix, Vit-D water solution and Vit-D NanoEm demonstrating the unexpected superior absorption properties of the Vit-D NanoEm in the spectral range of 230-520nm

The absorption tests revealed a surprising synergistic effect of Vit-D in a nano-emulsion which spans the wide spectral range of 220-540nm and is particularly pronounced at wavelengths of 240nm and above. This light spectral range covers both a wide UV range and the entire blue light spectral range.

As depicted in [0041], a clear distinction exists between the near negligible absorption of Vit-D in a water solution (overall and more particularly from 300nm and above) as compared to the absorption properties of the Vit-D nano-emulsion. [0041] also reveals that the Vit-D nanoemulsion has considerably higher absorption when compared to that of the Lipimix. The Vit-D Nano-emulsion maintains superiority at wavelengths above 240nm of the measured spectrum.

The effect of Vit-D addition to the Lipimix nano-emulsion was characterized in a sequential set of Vit-D nano-emulsions which were prepared by diluting each of the Vit-D nano-emulsions by half each time (See Table 7). As shown in [0040], the effects of Vit-D on absorption are observed over a broad range of 12.5-100 mg Vit-D/ml of Lipimix. The Vit-D dependent absorptive effects are particularly pronounced at a concentration of 100 mg/ml Vit-D, which is evident for nearly the entire spectral range (240-520nm). Moreover, these results are observed even with formulations diluted by adding water to achieve a diluted solution of 80% water

The results presented herein demonstrate an unexpected effect of Vitamin D addition to a Lipimix formulation. The addition of >10 mg/ml Vit-D (e.g., 12.5 mg/ml) to the nano-emulsion not only does not diminish the absorption spectra but surprisingly at Vit-D concentrations (>50 mg/ml) reinforces the UV to blue-light blocking effect in a synergistic fashion.

### Animal Models and Experimental Procedures

### Animal Model of Corneal Epithelium and Conjunctival Epithelium Damage

Blue light damage to the retina has been shown in studies examining acute and chronic exposure. Retina was found most sensitive to light at the shorter wavelengths (maximum sensitivity shown at 441 nm). Retinal photochemical injury from blue light has been demonstrated both in vitro and in vivo. In vitro studies of human retinal cells have identified cellular signaling pathways that result in oxidative damage which increases the risk of retinal photodamage and contributes to the development of age-related maculopathy. Even at the low intensity utilized in display devices (such as, for example, computer monitors and TV screens), blue light can induce reactive oxygen species (ROS) production and apoptosis in retinal cells. This damage can be induced by commercial light emitting diodes (LEDs). Interestingly the blue component of white light has also been shown to cause a dysfunction of the blood retinal barrier at the retinal pigment epithelium.

A rabbit model (New Zealand white rabbits) for keratoconjunctivitis is known in the art and described, for example, by Gilbard et al. (1987, J. Inv. Ophthal. Vis. Sci., 28:225-228). Slight modifications to the model of Gilbard et al. (1987, *supra*) are described in U.S. Patent Number (USPN) 7,723,287. Briefly, surgery may be performed on anesthetized rabbits using a surgical microscope (Inami, Japan) that calls for excision of the plical fold over the eye, occlusion of the lacrimal duct, and peeling of the palpebral and bulbar conjunctiva. This surgery may be performed on one eye of 20 rabbits having an average age of 3 months and of either sex. Surgery and all subsequent treatments were done in accordance with ARVO rules for animal care in research. Tear film osmolarity is elevated by postoperative day 1. Corneal epithelial glycogen levels decline progressively and conjunctival goblet cell density decreases. These pathologies lead to corneal epithelium damage covering the entire corneal surface by the fifth postoperative day. In one embodiment, treatment of the damaged eyes may commence at the fifth postoperative day.

### Evaluation of Rabbit Cornea

Lesions in fluorescein stained corneas may be clinically evaluated by biomicroscopy using a slit lamp (Haag Streit, Switzerland) with cobalt filter illumination. Photography of the fluorescein staining may be taken with a slit lamp mounted camera (Topcon, Japan). At the end of each experiment, the rabbits are sacrificed and their corneas are excised, fixed in paraformaldehyde, and examined for epithelial lesions.

### Histological Examination

At the end of the treatment, the rabbits are sacrificed by a lethal dose of intravenously injected pentobarbitone. Their eyes were enucleated and the corneas fixed in 4% paraformaldehyde. Corneas are embedded in paraffin blocks, sectioned, and stained with hematoxylin-eosin for light microscope examination.

## Claims

1. An ophthalmic topical pharmaceutical composition comprising a phospholipid nano-emulsion comprising Vitamin D that ranges from 0.02-2% w/v and phospholipids that range from 0.1-5% w/v, and wherein emulsified particles have a particle size ranging from 100-300 nanometers in diameter and wherein the phospholipids and vitamin D are in a ratio of at least 3:1, and wherein a therapeutically effective amount of the pharmaceutical composition is sufficient to absorb light in a wavelength range of 220-520 nm.

2. The pharmaceutical composition according to claim 1, wherein the pharmaceutical composition does not comprise a liquefied or compressed gas propellant.

3. The pharmaceutical composition according to claim 1, wherein the composition is in the form of eye drops or eye salve, optionally further comprising ophthalmologically acceptable carrier.

4. The pharmaceutical composition according to claim 3 that is a hypotonic composition.

5. The pharmaceutical composition according to any one of claims 1-4 for use in reducing absorption of light in a wavelength range of 220-520 nm, wherein the pharmaceutical composition is having a longer corneal retention time and/or an enhanced corneal penetration.

6. The pharmaceutical composition according to any one of claims 1-5 for use in treating a condition of the eye.

7. The pharmaceutical composition according to claim 6, wherein the condition of the eye comprises at least one of inflammation of the eye, dry eye, or glaucoma.

8. The pharmaceutical composition according to claim 6 or 7, wherein the condition of the eye comprises a disorder of the anterior segment of the eye and/or a physical or a chemical damage to the to the corneal or conjunctival epithelia.

9. The pharmaceutical composition of any one of claims 1-4 for use in storing or maintaining isolated corneas.

10. An ophthalmic pharmaceutical composition comprising Vitamin D a phospholipid nano-emulsion comprising Vitamin D that ranges from 0.02-2% wt/v and phospholipids **that** range from 0.1-5% w/v and wherein emulsified particles have a particle size ranging from 100-300 nanometers in diameter and wherein phospholipids and Vitamin D are in a ratio of at least 3:1, and wherein a therapeutically effective amount of the pharmaceutical composition is sufficient to deliver the Vitamin D to corneal tissue of an eye for a prolonged duration, wherein the prolonged duration is at least 1-10 minutes.

11. The pharmaceutical composition according to claim 10, wherein the pharmaceutical composition does not comprise a liquefied or compressed gas propellant.

12. The pharmaceutical composition according to claim 11, wherein the prolonged duration is at least 1 min and is less than 12 min.

13. The pharmaceutical composition according to any one of claims 10-12, wherein the composition is in the form of eye drops or eye salve, optionally further comprising ophthalmologically acceptable carrier.

14. The pharmaceutical composition according to any one of claims 10-13 for use in a method of delivering the Vitamin D to the eyes..

15. The pharmaceutical composition according to any one of claims 10-14 for use in a method of treating a condition of the eye.

## Patentansprüche

1. Ophthalmische topische pharmazeutische Zusammensetzung, umfassend eine Phospholipid-Nanoemulsion, die Vitamin D im Bereich von 0,02 bis 2% w/v und Phospholipide im Bereich von 0,1 bis 5% w/v umfasst, und wobei emulgierte Partikel eine Partikelgröße im Bereich von 100 bis 300 Nanometern im Durchmesser aufweisen und wobei die Phospholipide und Vitamin D in einem Verhältnis von mindestens 3:1 vorliegen und wobei eine therapeutisch wirksame Menge der pharmazeutischen Zusammensetzung ausreicht, um Licht in einem Wellenlängenbereich von 220 bis 520 nm zu absorbieren.

2. Pharmazeutische Zusammensetzung nach Anspruch 1, wobei die pharmazeutische Zusammensetzung kein verflüssigtes oder komprimiertes Gastreibmittel umfasst.

3. Pharmazeutische Zusammensetzung nach Anspruch 1, wobei die Zusammensetzung in Form von Augentropfen oder Augensalbe vorliegt, gegebenenfalls weiterhin umfassend einen ophthalmologisch akzeptablen Träger.

4. Pharmazeutische Zusammensetzung nach Anspruch 3, die eine hypotone Zusammensetzung ist.

5. Pharmazeutische Zusammensetzung nach einem der Ansprüche 1-4 zur Verwendung zur Verringerung der Absorption von Licht in einem Wellenlängenbereich von 220 bis 520 nm, wobei die pharmazeutische Zusammensetzung eine längere Hornhautverweilzeit und/oder eine verbesserte Hornhautpenetration aufweist.

6. Pharmazeutische Zusammensetzung nach einem der Ansprüche 1-5 zur Verwendung bei der Behandlung einer Erkrankung des Auges.

7. Pharmazeutische Zusammensetzung nach Anspruch 6, wobei die Erkrankung des Auges mindestens eine Entzündung des Auges, ein trockenes Auge und/oder ein Glaukom umfasst.

8. Pharmazeutische Zusammensetzung nach Anspruch 6 oder 7, wobei die Erkrankung des Auges eine Erkrankung des vorderen Augensegments und/oder eine physikalische oder chemische Schädigung der Hornhaut- oder Bindehautepithelien umfasst.

9. Pharmazeutische Zusammensetzung nach einem der Ansprüche 1-4 zur Verwendung bei der Lagerung oder Aufrechterhaltung isolierter Hornhäute.

10. Ophthalmische pharmazeutische Zusammensetzung, umfassend Vitamin D, eine Phospholipid-Nanoemulsion, die Vitamin D im Bereich von 0,02 bis 2% w/v und Phospholipide im Bereich von 0,1 bis 5% w/v umfasst, und wobei emulgierte Partikel eine Partikelgröße im Bereich von 100 bis 300 Nanometern im Durchmesser aufweisen und wobei Phospholipide und Vitamin D in einem Verhältnis von mindestens 3:1 vorliegen und wobei eine therapeutisch wirksame Menge der pharmazeutischen Zusammensetzung ausreicht, um das Vitamin D für eine längere Dauer an das Hornhautgewebe eines Auges abzugeben, wobei die verlängerte Dauer mindestens 1-10 Minuten beträgt.

11. Pharmazeutische Zusammensetzung nach Anspruch 10, wobei die pharmazeutische Zusammensetzung kein verflüssigtes oder komprimiertes Gastreibmittel umfasst.

12. Pharmazeutische Zusammensetzung nach Anspruch 11, wobei die verlängerte Dauer mindestens 1 min und weniger als 12 min beträgt.

13. Pharmazeutische Zusammensetzung nach einem der Ansprüche 10-12, wobei die Zusammensetzung in Form von Augentropfen oder Augensalbe vorliegt, gegebenenfalls weiterhin umfassend einen ophthalmologisch akzeptablen Träger.

14. Pharmazeutische Zusammensetzung nach einem der Ansprüche 10-13 zur Verwendung in einem Verfahren zur Verabreichung von Vitamin D an die Augen.

15. Pharmazeutische Zusammensetzung nach einem der Ansprüche 10-14 zur Verwendung in einem Verfahren zur Behandlung einer Erkrankung des Auges.

## Revendications

1. Composition pharmaceutique topique ophtalmique comprenant une nano-émulsion phospholipidique comprenant de la vitamine D dans une plage de 0,02 à 2 % p/v et des phospholipides dans une plage de 0,1 à 5 % p/v, et dans laquelle les particules émulsifiées ont une taille de particule dans une plage de 100-300 nanomètres de diamètre et dans laquelle les phospholipides et la vitamine D sont présents en un rapport d'au moins 3:1, et dans laquelle une quantité thérapeutiquement efficace de la composition pharmaceutique est suffisante pour absorber la lumière dans une plage de longueurs d'onde de 220-520 nm.

2. Composition pharmaceutique selon la revendication 1, dans laquelle la composition pharmaceutique ne comprend pas de propulseur de gaz liquéfié ou comprimé.

3. Composition pharmaceutique selon la revendication 1, dans laquelle la composition est sous la forme de gouttes oculaires ou de baume oculaire, comprenant éventuellement en outre un support ophtalmologiquement acceptable.

4. Composition pharmaceutique selon la revendication 3, qui est une composition hypotonique.

5. Composition pharmaceutique selon l'une quelconque des revendications 1-4 pour une utilisation pour réduire l'absorption de lumière dans une plage de longueurs d'onde de 220-520 nm, dans laquelle la composition pharmaceutique a un temps de rétention cornéenne plus long et/ou une pénétration cornéenne améliorée.

6. Composition pharmaceutique selon l'une quelconque des revendications 1-5 pour une utilisation dans le traitement d'une affection de l'œil.

7. Composition pharmaceutique selon la revendication 6, dans laquelle l'affection de l'œil comprend au moins l'un parmi une inflammation de l'œil, une sécheresse oculaire ou un glaucome.

8. Composition pharmaceutique selon la revendication 6 ou 7, dans laquelle l'affection de l'œil comprend un trouble du segment antérieur de l'œil et/ou une lésion physique ou une lésion chimique de l'épithélium cornéen ou conjonctival.

9. Composition pharmaceutique selon l'une quelconque des revendications 1-4 pour une utilisation dans le stockage ou le maintien de cornées isolées.

10. Composition pharmaceutique ophtalmique comprenant une nano-émulsion phospholipidique comprenant de la vitamine D dans la plage de 0,02 à 2 % en p/v et des phospholipides dans la plage de 0,1 à 5 % en p/v et dans laquelle les particules émulsifiées ont une taille de particule dans la plage de 100-300 nanomètres de diamètre et dans laquelle les phospholipides et la vitamine D sont présents en un rapport d'au moins 3:1, et dans laquelle une quantité thérapeutiquement efficace de la composition pharmaceutique est suffisante pour délivrer la vitamine D à un tissu cornéen d'un œil pendant une durée prolongée, dans laquelle la durée prolongée est d'au moins 1-10 minutes.

11. Composition pharmaceutique selon la revendication 10, dans laquelle la composition pharmaceutique ne comprend pas de propulseur de gaz liquéfié ou comprimé.

12. Composition pharmaceutique selon la revendication 11, dans laquelle la durée prolongée est d'au moins 1 min et est inférieure à 12 min.

13. Composition pharmaceutique selon l'une quelconque des revendications 10 à 12, dans laquelle la composition est sous la forme de gouttes oculaires ou de baume oculaire, comprenant éventuellement en outre un support ophtalmologiquement acceptable.

14. Composition pharmaceutique selon l'une quelconque des revendications 10-13 pour une utilisation dans un procédé d'administration de la vitamine D aux yeux.

15. Composition pharmaceutique selon l'une quelconque des revendications 10-14 pour une utilisation dans un procédé de traitement d'une affection de l'œil.
